(19) Europäisches Patentamt
European Patent Office
Office européen des brevets

(11) EP 4 442 710 A1

(12) **EUROPEAN PATENT APPLICATION**
published in accordance with Art. 153(4) EPC

(43) Date of publication:
**09.10.2024 Bulletin 2024/41**

(21) Application number: **22900602.8**

(22) Date of filing: **01.12.2022**

(51) International Patent Classification (IPC):
***C07K 16/28*** *(2006.01)* ***A61P 35/00*** *(2006.01)*
***A61K 39/395*** *(2006.01)*

(52) Cooperative Patent Classification (CPC):
**A61K 39/395; A61P 35/00; C07K 16/28**

(86) International application number:
**PCT/CN2022/135865**

(87) International publication number:
**WO 2023/098798 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.12.2021 CN 202111452473**

(71) Applicant: **Chia Tai Tianqing Pharmaceutical Group Co., Ltd**
**Lianyungang, Jiangsu 222062 (CN)**

(72) Inventors:
- **SHEN, Lili**
  **Nanjing City, Jiangsu 211100 (CN)**
- **ZHANG, Xiquan**
  **Nanjing City, Jiangsu 211100 (CN)**
- **WANG, Xunqiang**
  **Nanjing City, Jiangsu 211100 (CN)**
- **YU, Ding**
  **Nanjing City, Jiangsu 211100 (CN)**
- **LIU, Lu**
  **Lianyungang, Jiangsu 222062 (CN)**

(74) Representative: **WSL Patentanwälte Partnerschaft mbB**
**Kaiser-Friedrich-Ring 98**
**65185 Wiesbaden (DE)**

Remarks:
The complete document including Reference Table(s) and the Sequence Listing(s) can be downloaded from the EPO website

(54) **DRUG COMBINATION FOR TREATING NON-SMALL CELL LUNG CANCER**

(57) Provided is a drug combination for treating non-small cell lung cancer, the combination comprising an anti-PD-L1 antibody and a chemotherapeutic drug, and optionally anlotinib or a pharmaceutically acceptable salt thereof. Further provided is the use of the drug combination in the preparation of a drug for treating non-small cell lung cancer.

EP 4 442 710 A1

## Description

### TECHNICAL FIELD

The present application belongs to the field of biopharmaceuticals, and particularly relates to a pharmaceutical combination for use in treating non-small cell lung cancer.

### BACKGROUND

According to the Global Cancer Statistics (2020 edition), lung cancer is the second most common cancer worldwide after breast cancer, and still the cancer causing the greatest number of deaths worldwide. In China, lung cancer ranks first among malignant tumors in terms of incidence and mortality. In 2020, there were about 4.57 million new cases of cancer in China, of which about 820,000 were lung cancer, accounting for about 17.9%. There are about 3 million cancer deaths in China, of which about 710,000 are deaths due to lung cancer, accounting for about 23.8%.

Non-small cell lung cancer (NSCLC) cases account for 85% of all lung cancer cases, most of which are in the advanced stage at the first diagnosis, with a 5-year survival rate of only 16% despite the active intervention of traditional therapies such as surgery, chemotherapy, radiotherapy, and targeted therapy.

Currently, despite numerous treatment options for patients with non-small cell lung cancer, there is still a need for more effective therapeutic agents for clinical use, particularly a combination of more than one drug.

### SUMMARY

In one aspect, the present application provides a pharmaceutical combination for use in treating non-small cell lung cancer, comprising: an anti-PD-L1 antibody and at least one chemotherapeutic drug.

Further, the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

In some embodiments, the chemotherapeutic drug includes, but is not limited to, one or more of a platinum-based anti-tumor drug, a taxane anti-tumor drug, an antimetabolite anti-tumor drug, a camptothecin anti-tumor drug, a nitrogen mustard anti-tumor drug, an anthracycline anti-tumor drug, a vinblastine anti-tumor drug, a podophyllotoxin anti-tumor drug, and a hormone anti-tumor drug.

In some embodiments, the chemotherapeutic drug is selected from the group consisting of one or more of a platinum-based anti-tumor drug, a taxane anti-tumor drug, and an antimetabolite anti-tumor drug. In some embodiments, the chemotherapeutic drug includes: a platinum-based anti-tumor drug; and at least one selected from the group consisting of a taxane anti-tumor drug and an antimetabolite anti-tumor drug. In some embodiments, the chemotherapeutic drug is a platinum-based anti-tumor drug and/or a taxane anti-tumor drug. In some embodiments, the chemotherapeutic drug is a platinum-based anti-tumor drug and/or an antimetabolite anti-tumor drug.

In some embodiments of the present application, the pharmaceutical combination comprises: an anti-PD-L1 antibody and at least one chemotherapeutic drug, including a platinum-based anti-tumor drug. In some embodiments, the pharmaceutical combination comprises: an anti-PD-L1 antibody and at least one platinum-based anti-tumor drug.

Further, the platinum-based anti-tumor drug is selected from the group consisting of one or more of cisplatin, carboplatin, nedaplatin, dicycloplatin, picoplatin, oxaliplatin, miriplatin, lobaplatin, triplatin tetranitrate, phenanthriplatin, and satraplatin; preferably, the platinum-based anti-tumor drug is carboplatin and/or cisplatin; more preferably, the platinum-based anti-tumor drug is carboplatin. In some embodiments, the taxane anti-tumor drug is selected from the group consisting of one or more of paclitaxel, paclitaxel liposome, albumin-bound paclitaxel, and docetaxel; preferably, the taxane anti-tumor drug is paclitaxel and/or docetaxel and/or paclitaxel liposome; more preferably, the taxane anti-tumor drug is paclitaxel. In some embodiments, the antimetabolite anti-tumor drug is selected from the group consisting of one or more of fluorouracils, cytosines, purines, and antifolates; in some embodiments, the antimetabolite anti-tumor drug is selected from the group consisting of one or more of carmofur, 5-fluorouracil, tegafur, capecitabine, tegafur-gimeracil-oteracil potassium, difuradin, doxifluridine, trifluridine, cytarabine, gemcitabine, azacitidine, ancitabine, mercaptopurine, fludarabine, methotrexate, and pemetrexed; preferably the antimetabolite anti-tumor drug is selected from the group consisting of pemetrexed and/or gemcitabine; more preferably, the antimetabolite anti-tumor drug is pemetrexed.

In some embodiments, the chemotherapeutic drug includes: (1) one or more selected from the group consisting of cisplatin, carboplatin, nedaplatin, dicycloplatin, picoplatin, oxaliplatin, miriplatin, lobaplatin, lobaplatin, triplatin tetranitrate, phenanthriplatin, and satraplatin, and (2) at least one selected from the group consisting of a taxane anti-tumor drug and an antimetabolite anti-tumor drug, the taxane anti-tumor drug being selected from the group consisting of one or more of paclitaxel, paclitaxel liposome, albumin-bound paclitaxel, and docetaxel, and the antimetabolite anti-tumor drug being selected from the group consisting of one or more of carmofur, 5-fluorouracil, tegafur, capecitabine, tegafur-gimeracil-oteracil potassium, difuradin, doxifluridine, trifluridine, cytarabine, gemcitabine, azacitidine, ancitabine, mercaptopurine, fludarabine, methotrexate, and pemetrexed. In some embodiments, the chemotherapeutic drug includes a platinum-based anti-tumor drug. In some embodiments, the chemotherapeutic drug includes: one or more selected from the group consisting of cisplatin, carboplatin, nedaplatin, dicycloplatin, picoplatin, oxaliplatin, miriplatin, lobaplatin, lobaplatin, triplatin tetranitrate, phenanthriplatin, and satraplatin.

In some embodiments, the chemotherapeutic drug is a platinum-based anti-tumor drug and paclitaxel. In some embodiments, the chemotherapeutic drug is carboplatin and a taxane anti-tumor drug. In some embodiments, the chemotherapeutic drug is carboplatin and paclitaxel. In some embodiments, the chemotherapeutic drug is cisplatin and paclitaxel. In some embodiments, the chemotherapeutic drug is a platinum-based anti-tumor drug and pemetrexed. In some embodiments, the chemotherapeutic drug is carboplatin and an antimetabolite anti-tumor drug. In some embodiments, the chemotherapeutic drug is carboplatin and pemetrexed. In some embodiments, the chemotherapeutic drug is cisplatin and pemetrexed.

In some embodiments, the chemotherapeutic drug is one or more of carboplatin, pemetrexed, and paclitaxel.

In some embodiments, the pharmaceutical combination further comprises anlotinib or a pharmaceutically acceptable salt thereof.

In another aspect, the present application provides a pharmaceutical combination for use in treating non-small cell lung cancer, comprising a first pharmaceutical combination and optionally a second pharmaceutical combination. In some embodiments, the pharmaceutical combination comprises a first pharmaceutical combination administered to a patient in need thereof in a first treatment phase, and optionally a second pharmaceutical combination administered to the patient in need thereof in a second treatment phase. In some embodiments, the first treatment phase comprises 1 to 10 treatment cycles, preferably 2 to 8 treatment cycles, further preferably 4 to 6 treatment cycles, and most preferably 4 treatment cycles. In some embodiments, the pharmaceutical combination comprises a first pharmaceutical combination administered to a patient in need thereof in a first treatment phase, and a second pharmaceutical combination administered to the patient in need thereof in a second treatment phase, the second treatment phase being performed sequentially after the first treatment phase.

In some embodiments, the pharmaceutical combination comprises: (1) a first pharmaceutical combination comprising an anti-PD-L1 antibody and at least one chemotherapeutic drug, administered to a patient in need thereof in a first treatment phase, and (2) a second pharmaceutical combination comprising an anti-PD-L1 antibody, anlotinib or a pharmaceutically acceptable salt thereof, and optionally a chemotherapeutic drug, administered to the patient in need thereof in a second treatment phase.

In some embodiments, the pharmaceutical combination comprises: (1) a first pharmaceutical combination comprising an anti-PD-L1 antibody and at least one of a platinum-based anti-tumor drug, a taxane anti-tumor drug, and an antimetabolite anti-tumor drug, administered to a patient in need thereof in a first treatment phase, and (2) a second pharmaceutical combination comprising an anti-PD-L1 antibody, anlotinib or a pharmaceutically acceptable salt thereof, and optionally a chemotherapeutic drug, administered to the patient in need thereof in a second treatment phase.

In some embodiments, the pharmaceutical combination comprises: (1) a first pharmaceutical combination comprising an anti-PD-L1 antibody and a platinum-based anti-tumor drug, administered to a patient in need thereof in a first treatment phase, and (2) a second pharmaceutical combination comprising an anti-PD-L1 antibody, anlotinib or a pharmaceutically acceptable salt thereof, and optionally a chemotherapeutic drug, administered to the patient in need thereof in a second treatment phase.

In some embodiments, the pharmaceutical combination comprises: (1) an anti-PD-L1 antibody and a chemotherapeutic drug comprising a platinum-based anti-tumor drug administered to a patient in need thereof, and sequentially administered (2) an anti-PD-L1 antibody, anlotinib or a pharmaceutically acceptable salt thereof, and optionally a chemotherapeutic drug.

In some embodiments, the pharmaceutical combination comprises: (1) a first pharmaceutical combination comprising an anti-PD-L1 antibody and a platinum-based anti-tumor drug, administered to a patient in need thereof in a first treatment phase, and (2) a second pharmaceutical combination comprising an anti-PD-L1 antibody, anlotinib or a pharmaceutically acceptable salt thereof, and an antimetabolite anti-tumor drug, administered to the patient in need thereof in a second treatment phase.

In some embodiments, the pharmaceutical combination comprises: (1) a first pharmaceutical combination comprising an anti-PD-L1 antibody and a platinum-based anti-tumor drug, administered to a patient in need thereof in a first treatment phase, and (2) a second pharmaceutical combination comprising an anti-PD-L1 antibody, anlotinib or

a pharmaceutically acceptable salt thereof, and pemetrexed, administered to the patient in need thereof in a second treatment phase.

In some embodiments, the pharmaceutical combination comprises: (1) an anti-PD-L1 antibody and a chemotherapeutic drug comprising a platinum-based anti-tumor drug, administered to a patient in need thereof, and sequentially administered (2) an anti-PD-L1 antibody, anlotinib or a pharmaceutically acceptable salt thereof, and an antimetabolite anti-tumor drug.

In some embodiments, the pharmaceutical combination comprises: (1) an anti-PD-L1 antibody and a chemotherapeutic drug comprising a platinum-based anti-tumor drug administered to a patient in need thereof, and sequentially administered (2) an anti-PD-L1 antibody, anlotinib or a pharmaceutically acceptable salt thereof, and pemetrexed.

In yet another aspect, the present application provides a pharmaceutical combination for use in treating squamous non-small cell lung cancer, comprising a first pharmaceutical combination and optionally a second pharmaceutical combination, wherein the first pharmaceutical combination comprises an anti-PD-L1 antibody and at least one chemotherapeutic drug. In some embodiments, the chemotherapeutic drug in the first pharmaceutical combination is selected from the group consisting of one or more of a platinum-based anti-tumor drug, a taxane anti-tumor drug, and an antimetabolite anti-tumor drug. In some embodiments, the chemotherapeutic drug in the first pharmaceutical combination includes: a platinum-based anti-tumor drug; and at least one selected from the group consisting of a taxane anti-tumor drug and an antimetabolite anti-tumor drug. In some embodiments, the chemotherapeutic drug in the first pharmaceutical combination is selected from the group consisting of one or more of carboplatin, pemetrexed, and paclitaxel. In some embodiments, the chemotherapeutic drug in the first pharmaceutical combination is a platinum-based anti-tumor drug and a taxane anti-tumor drug. In some embodiments, the chemotherapeutic drug in the first pharmaceutical combination is a platinum-based anti-tumor drug and paclitaxel. In some embodiments, the chemotherapeutic drug in the first pharmaceutical combination is carboplatin and a taxane anti-tumor drug. In some embodiments, the chemotherapeutic drug in the first pharmaceutical combination is carboplatin and paclitaxel.

In some embodiments, the second pharmaceutical combination comprises an anti-PD-L1 antibody and anlotinib or a pharmaceutically acceptable salt thereof. In some embodiments, the second pharmaceutical combination further comprises a chemotherapeutic drug. In some embodiments, the chemotherapeutic drug in the second pharmaceutical combination is selected from the group consisting of one or more of a platinum-based anti-tumor drug, a taxane anti-tumor drug, and an antimetabolite anti-tumor drug. In some embodiments, the chemotherapeutic drug in the second pharmaceutical combination is selected from the group consisting of one or more of carboplatin, pemetrexed, and paclitaxel. In some embodiments, the chemotherapeutic drug in the second pharmaceutical combination is a taxane anti-tumor drug. In some embodiments, the chemotherapeutic drug in the second pharmaceutical combination is paclitaxel.

In some specific embodiments, the pharmaceutical combination comprises a first pharmaceutical combination administered to a patient in need thereof in a first treatment phase, and optionally a second pharmaceutical combination administered to the patient in need thereof in a second treatment phase, wherein the first pharmaceutical combination comprises an anti-PD-L1 antibody, carboplatin, and paclitaxel, and the second pharmaceutical combination comprises an anti-PD-L1 antibody and anlotinib or a pharmaceutically acceptable salt thereof. It can be understood that the second treatment phase is performed sequentially after the first treatment phase.

In some embodiments, the anti-PD-L1 antibody, carboplatin, and paclitaxel comprised in the first pharmaceutical combination are administered to a patient in need thereof, wherein the anti-PD-L1 antibody is administered at a daily dose of 600-2400 mg (e.g., 800 mg, 1200 mg, 1600 mg, or 2000 mg), paclitaxel is administered at a daily dose of 100-200 $mg/m^2$ (e.g., 125 $mg/m^2$, 150 $mg/m^2$, or 175 $mg/m^2$), and carboplatin is administered at a dose of AUC 1-10 mg/mL/min (e.g., AUC 3 mg/mL/min, AUC 5 mg/mL/min, or AUC 8 mg/mL/min) with a daily dose of no more than 800 mg.

In some embodiments, the anti-PD-L1 antibody and anlotinib or the pharmaceutically acceptable salt thereof comprised in the second pharmaceutical combination are administered to a patient in need thereof, wherein the anti-PD-L1 antibody is administered at a daily dose of 600-2400 mg (e.g., 800 mg, 1200 mg, 1600 mg, or 2000 mg), and anlotinib or the pharmaceutically acceptable salt thereof is administered at a daily dose of 6-12 mg (e.g., 8 mg or 10 mg).

In some embodiments, the pharmaceutical combination further comprises a pharmaceutically acceptable carrier.

In yet another aspect, the present application provides a pharmaceutical combination for use in treating non-squamous non-small cell lung cancer or lung adenocarcinoma, comprising a first pharmaceutical combination, and optionally a second pharmaceutical combination, wherein, in some embodiments, the chemotherapeutic drug in the first pharmaceutical combination is selected from the group consisting of one or more of a platinum-based anti-tumor drug, a taxane anti-tumor drug, and an antimetabolite anti-tumor drug. In some embodiments, the chemotherapeutic drug in the first pharmaceutical combination is selected from the group consisting of one or more of carboplatin, pemetrexed, and paclitaxel. In some embodiments, the chemotherapeutic drug in the first pharmaceutical combination is a platinum-based anti-tumor drug and pemetrexed. In some embodiments, the chemotherapeutic drug in the first pharmaceutical combination is carboplatin and an antimetabolite anti-tumor drug. In some embodiments, the chemotherapeutic drug in

the first pharmaceutical combination is carboplatin and pemetrexed.

In some embodiments, the second pharmaceutical combination comprises an anti-PD-L1 antibody and anlotinib or a pharmaceutically acceptable salt thereof. In some embodiments, the second pharmaceutical combination further comprises a chemotherapeutic drug. In some embodiments, the chemotherapeutic drug in the second pharmaceutical combination is selected from the group consisting of one or more of a platinum-based anti-tumor drug, a taxane anti-tumor drug, and an antimetabolite anti-tumor drug. In some embodiments, the chemotherapeutic drug in the second pharmaceutical combination is selected from the group consisting of one or more of carboplatin, pemetrexed, and paclitaxel. In some embodiments, the chemotherapeutic drug in the second pharmaceutical combination is an antimetabolite anti-tumor drug. In some embodiments, the chemotherapeutic drug in the second pharmaceutical combination is pemetrexed and/or gemcitabine. In some embodiments, the chemotherapeutic drug in the second pharmaceutical combination is pemetrexed.

In some specific embodiments, the pharmaceutical combination comprises a first pharmaceutical combination administered to a patient in need thereof in a first treatment phase and a second pharmaceutical combination administered to the patient in need thereof in a second treatment phase, wherein the first pharmaceutical combination comprises an anti-PD-L1 antibody, carboplatin, and pemetrexed, and the second pharmaceutical combination comprises an anti-PD-L1 antibody, anlotinib or a pharmaceutically acceptable salt thereof, and pemetrexed. It can be understood that the second treatment phase is performed sequentially after the first treatment phase.

In some embodiments, the anti-PD-L1 antibody, carboplatin, and pemetrexed comprised in the first pharmaceutical combination are administered to a patient in need thereof, wherein the anti-PD-L1 antibody is administered at a daily dose of 600-2400 mg (e.g., 800 mg, 1200 mg, 1600 mg, or 2000 mg), pemetrexed is administered at a daily dose of 200-800 mg/$m^2$ (e.g., 300 mg/$m^2$, 400 mg/$m^2$, 500 mg/$m^2$, 600 mg/$m^2$, or 700 mg/$m^2$), and carboplatin is administered at a dose of AUC 1-10 mg/mL/min (e.g., AUC 3 mg/mL/min, AUC 5 mg/mL/min, or AUC 8 mg/mL/min) with a daily dose of no more than 800 mg.

In some embodiments, the anti-PD-L1 antibody, anlotinib or the pharmaceutically acceptable salt thereof, and pemetrexed comprised in the second pharmaceutical combination are administered to a patient in need thereof, wherein the anti-PD-L1 antibody is administered at a daily dose of 600-2400 mg (e.g., 800 mg, 1200 mg, 1600 mg, or 2000 mg), anlotinib or the pharmaceutically acceptable salt thereof is administered at a daily dose of 6-12 mg (e.g., 8 mg or 10 mg), and pemetrexed is administered at a daily dose of 200-800 mg/$m^2$ (e.g., 300 mg/$m^2$, 400 mg/$m^2$, 500 mg/$m^2$, 600 mg/$m^2$, or 700 mg/$m^2$).

In some embodiments, the pharmaceutical combination further comprises a pharmaceutically acceptable carrier.

In some embodiments, in the pharmaceutical combination comprising the anti-PD-L1 antibody and at least one chemotherapeutic drug described herein, the anti-PD-L1 antibody, at least one chemotherapeutic drug, and optionally anlotinib or a pharmaceutically acceptable salt thereof are each in the form of a pharmaceutical composition, which can be administered simultaneously, successively, or sequentially. In some embodiments, the pharmaceutical combination comprises a pharmaceutical composition comprising the anti-PD-L1 antibody, a pharmaceutical composition comprising the chemotherapeutic drug, and optionally a pharmaceutical composition comprising anlotinib or the pharmaceutically acceptable salt thereof.

Further, the pharmaceutical combinations described above of the present application are packaged in the same kit, which further comprises instructions for the treatment of non-small cell lung cancer.

In still another aspect, the present application provides a kit for use in treating non-small cell lung cancer, comprising: an anti-PD-L1 antibody, at least one chemotherapeutic drug, and optionally anlotinib or a pharmaceutically acceptable salt thereof. In some embodiments, the anti-PD-L1 antibody is contained in a first compartment, the optional anlotinib or the pharmaceutically acceptable salt thereof is contained in a second compartment, and the chemotherapeutic drug is contained in an additional compartment. Optionally, the number of compartments in the kit can be increased as appropriate depending on the number of chemotherapeutic drugs. The components can be administered to a patient in need simultaneously, successively, or sequentially. In some embodiments, the kit further comprises instructions for treating non-small cell lung cancer. In some embodiments, the kit comprises: a pharmaceutical composition comprising the anti-PD-L1 antibody, a pharmaceutical composition comprising the chemotherapeutic drug, and optionally a pharmaceutical composition comprising anlotinib or the pharmaceutically acceptable salt thereof.

Further, the kit described above is suitable for administration within a single treatment cycle (e.g., a treatment cycle of 21 days), and comprises a pharmaceutical composition comprising 600-2400 mg of the anti-PD-L1 antibody. In some embodiments, the kit is suitable for administration within a single treatment cycle (e.g., a treatment cycle of 21 days), and comprises a pharmaceutical composition comprising 600-2400 mg of the anti-PD-L1 antibody, and optionally a pharmaceutical composition comprising 84-168 mg of anlotinib or the pharmaceutically acceptable salt thereof. In some embodiments, every 1 week, every 2 weeks, every 3 weeks, or every 4 weeks is counted as one treatment cycle. In a preferred embodiment, every 3 weeks is counted as one treatment cycle.

The amount of the anti-PD-L1 antibody administered can be determined according to the severity of the disease,

the response of the disease, any treatment-related toxicity, and the age and health of the patient. For example, the daily dose of the anti-PD-L1 antibody administered may be 600-2400 mg. In some embodiments, the daily dose of the anti-PD-L1 antibody administered may be 600 mg, 800 mg, 1000 mg, 1200 mg, 1400 mg, 1600 mg, 1800 mg, 2000 mg, 2200 mg, or 2400 mg. In some embodiments, the anti-PD-L1 antibody is administered parenterally; in some embodiments, the anti-PD-L1 antibody is administered intravenously; in some embodiments, the concentration of the anti-PD-L1 antibody in the pharmaceutical composition comprising the anti-PD-L1 antibody is 10-60 mg/mL; in some embodiments, the concentration of the anti-PD-L1 antibody in the pharmaceutical composition comprising the anti-PD-L1 antibody is 10 mg/mL, 20 mg/mL, 30 mg/mL, 40 mg/mL, 50 mg/mL, or 60 mg/mL.

The administration regimen for the anti-PD-L1 antibody can be determined comprehensively depending on the activity and toxicity of the drug, the tolerance of a patient, and the like. In some embodiments, for the anti-PD-L1 antibody, every 1 week, every 2 weeks, every 3 weeks, or every 4 weeks is counted as one treatment cycle; in some embodiments, the anti-PD-L1 antibody is administered once every week, every 2 weeks, every 3 weeks, or every 4 weeks. In some embodiments, the anti-PD-L1 antibody is at a dose of 600-2400 mg in each treatment cycle; in some embodiments, the anti-PD-L1 antibody is at a dose of 1200 mg in each treatment cycle. In some embodiments, the anti-PD-L1 antibody is administered once every 3 weeks at a dose of 600-2400 mg each time. In some embodiments, anlotinib or the pharmaceutically acceptable salt thereof is administered at a daily dose of 6 mg, 8 mg, 10 mg, or 12 mg on an administration regimen of consecutively 2-week treatment and then 1-week interruption.

In some embodiments, the pharmaceutical combination comprising the anti-PD-L1 antibody and at least one chemotherapeutic drug described herein comprises a pharmaceutical composition comprising the anti-PD-L1 antibody and a pharmaceutical composition comprising the chemotherapeutic drug, wherein the pharmaceutical composition comprising the anti-PD-L1 antibody is prepared in a unit-dose or multiple-dose form suitable for administering to a patient 600-2400 mg of the anti-PD-L1 antibody at a first dose.

In some embodiments, the pharmaceutical combination comprising the anti-PD-L1 antibody and at least one chemotherapeutic drug described herein further comprises a pharmaceutical composition comprising anlotinib or the pharmaceutically acceptable salt thereof, wherein the pharmaceutical composition comprising anlotinib or the pharmaceutically acceptable salt thereof is prepared in a unit-dose form suitable for administering to a patient 6 mg, 8 mg, 10 mg, and/or 12 mg of anlotinib or the pharmaceutically acceptable salt thereof daily for 14 consecutive days.

In some embodiments, the pharmaceutical combination comprising the anti-PD-L1 antibody and at least one chemotherapeutic drug described herein comprises: a pharmaceutical composition comprising 1200 mg of the anti-PD-L1 antibody provided in a multiple-dose form. In some embodiments, the pharmaceutical combination comprising the anti-PD-L1 antibody and the chemotherapeutic drug described herein further comprises: a pharmaceutical composition comprising anlotinib or the pharmaceutically acceptable salt thereof in a unit dose of 8 mg, 10 mg, and/or 12 mg.

Further, the pharmaceutical combination comprising the anti-PD-L1 antibody and at least one chemotherapeutic drug described herein is a formulation suitable for administration in a single treatment cycle (e.g., one treatment cycle of 21 days), comprising a pharmaceutical composition comprising 600-2400 mg of the anti-PD-L1 antibody. In some embodiments, the pharmaceutical combination comprising the anti-PD-L1 antibody and at least one chemotherapeutic drug described herein further comprises a pharmaceutical composition comprising 84-168 mg of anlotinib or the pharmaceutically acceptable salt thereof.

Further, the pharmaceutical combination comprising the anti-PD-L1 antibody, the chemotherapeutic drug, and anlotinib or the pharmaceutically acceptable salt thereof described herein comprises the anti-PD-L1 antibody and anlotinib in a weight ratio of (0.35-29):1, preferably (3.5-29):1, more preferably (3.5-14.5):1, and most preferably (7-14.5):1. The anti-PD-L1 antibody and anlotinib are packaged either separately or together. Anlotinib can be packaged in multiple aliquots (e.g., 2 aliquots, 7 aliquots, 14 aliquots, 28 aliquots, or more); the anti-PD-L1 antibody can be packaged in a single aliquot or multiple aliquots (e.g., 2 aliquots, 4 aliquots, or more).

In still another aspect, the present application further provides use of the pharmaceutical combination of the present application or the kit of the present application for preparing a medicament for use in treating non-small cell lung cancer in a patient. Alternatively, the present application further provides a method for treating non-small cell lung cancer, comprising administering to a patient in need thereof an effective amount of the pharmaceutical combination of the present application or the kit of the present application. Alternatively, the present application further provides use of the pharmaceutical combination of the present application or the kit of the present application for treating non-small cell lung cancer in a patient. Alternatively, the present application further provides the pharmaceutical combination described above or the kit of the present invention for use in treating non-small cell lung cancer in a patient.

In some embodiments, the method for treating non-small cell lung cancer provided herein comprises administering to a patient in need thereof a therapeutically effective amount of the anti-PD-L1 antibody and at least one chemotherapeutic drug. In some embodiments, the method comprises administering to a patient in need thereof a first pharmaceutical combination in a first treatment phase; and optionally administering to the patient in need thereof a second pharmaceutical combination in a second treatment phase. In some embodiments, the first pharmaceutical combination comprises an anti-PD-L1 antibody and at least one chemotherapeutic drug. In some embodiments, the second pharmaceutical combination comprises an anti-PD-L1 antibody, anlotinib or a pharmaceutically acceptable salt thereof, and optionally a chemotherapeutic drug.

In some specific embodiments, the method for treating squamous non-small cell lung cancer provided herein comprises administering to a patient in need thereof a first pharmaceutical combination in a first treatment phase and administering to the patient in need thereof a second pharmaceutical combination in a second treatment phase, wherein the first pharmaceutical combination comprises an anti-PD-L1 antibody, carboplatin, and paclitaxel, and the second pharmaceutical combination comprises an anti-PD-L1 antibody and anlotinib or a pharmaceutically acceptable salt thereof. It can be understood that the second treatment phase is performed sequentially after the first treatment phase.

In some other specific embodiments, the method for treating non-squamous non-small cell lung cancer or lung adenocarcinoma provided herein comprises administering to a patient in need thereof a first pharmaceutical combination in a first treatment phase and administering the patient in need thereof a second pharmaceutical combination in a second treatment phase, wherein the first pharmaceutical combination comprises an anti-PD-L1 antibody, carboplatin, and pemetrexed, and the second pharmaceutical combination comprises an anti-PD-L1 antibody, anlotinib or a pharmaceutically acceptable salt thereof, and pemetrexed. It can be understood that the second treatment phase is performed sequentially after the first treatment phase.

In some embodiments, the first treatment phase comprises 1 to 10 treatment cycles, preferably 2 to 8 treatment cycles, further preferably 4 to 6 treatment cycles, and most preferably 4 treatment cycles.

Further, the anti-PD-L1 antibody, the chemotherapeutic drug, and anlotinib or the pharmaceutically acceptable salt thereof are each in the form of a pharmaceutical composition, and can be administered simultaneously, sequentially, or intermittently. Still further, the anti-PD-L1 antibody is administered once every week, every 2 weeks, every 3 weeks, or every 4 weeks; further, the anti-PD-L1 antibody is administered once every 3 weeks; preferably, the anti-PD-L1 antibody is administered at a dose of 600-2400 mg each time; further, the anti-PD-L1 antibody is administered at a dose of 1200 mg each time. Still further, anlotinib is administered at a dose of 6 mg, 8 mg, 10 mg, or 12 mg once daily on an administration regimen of consecutively 2-week treatment and then 1-week interruption.

Anlotinib or Pharmaceutically Acceptable Salt Thereof

Anlotinib, with a chemical name of 1-[[[4-(4-fhioro-2-methyl-1*H*-indol-5-yl)oxy-6-methoxyquinolin-7-yl]oxy] methyl]cyclopropylamine, has the following structural formula:

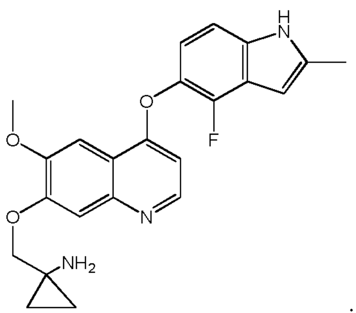

.

The pharmaceutically acceptable salt of anlotinib includes, but is not limited to salts formed from anlotinib and an acid selected from the group consisting of the following: hydrochloric acid, hydrobromic acid, sulfuric acid, nitric acid, phosphoric acid, acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, p-toluenesulfonic acid, 3-phenylpropionic acid, trimethylacetic acid, t-butylacetic acid, dodecyl sulfuric acid, gluconic acid, glutamic acid, hydroxynaphthoic acid, salicylic acid, and stearic acid; in some embodiments, the pharmaceutically acceptable salt is a hydrochloride salt or a maleate salt; in some embodiments, the pharmaceutically acceptable salt is a dihydrochloride salt.

Unless otherwise stated, the dose of anlotinib or the pharmaceutically acceptable salt thereof referred to in the present application is based on the molecular weight of the free base of anlotinib.

Anlotinib or the pharmaceutically acceptable salt thereof can be administered through various routes including gastrointestinal and parenteral routes, including, but not limited to, oral, intravenous, intra-arterial, transdermal, sublingual, intramuscular, rectal, transbuccal, intranasal, inhalational, vaginal, intraocular, local, subcutaneous, intra-adipose, intra-articular, intraperitoneal, and intrathecal administrations. In some specific embodiments, anlotinib or the pharmaceutically acceptable salt thereof is administered orally. The amount of anlotinib or the pharmaceutically acceptable salt thereof administered can be determined according to the severity of the disease, the response of the disease, any

treatment-related toxicity, and the age and health of the patient. For example, anlotinib or the pharmaceutically acceptable salt thereof can be administered at a daily dose of 2 mg to 20 mg; in some embodiments, anlotinib or the pharmaceutically acceptable salt thereof can be administered at a daily dose of 2 mg, 3 mg, 4 mg, 5 mg, 6 mg, 7 mg, 8 mg, 9 mg, 10 mg, 11 mg, 12 mg, 13 mg, 14 mg, 15 mg, or 16 mg. Anlotinib or the pharmaceutically acceptable salt thereof can be administered once or multiple times daily. In some embodiments, anlotinib or the pharmaceutically acceptable salt thereof is administered once daily in the form of an oral solid formulation.

The administration regimen for anlotinib or the pharmaceutically acceptable salt thereof can be determined comprehensively depending on the activity and toxicity of the drug, the tolerance of the patient, and the like. Preferably, anlotinib or the pharmaceutically acceptable salt thereof is administered intermittently. The intermittent administration comprises a treatment period and an interruption period. Anlotinib or the pharmaceutically acceptable salt thereof can be administered once or multiple times daily in the treatment period. For example, the ratio of the treatment period to the interruption period in days is 2:(0.5-5), 2:(0.5-3), 2:(0.5-2), or 2:(0.5-1). In some embodiments, anlotinib or the pharmaceutically acceptable salt thereof is administered on a regimen of consecutively 2-week treatment and then 2-week interruption. In some embodiments, anlotinib or the pharmaceutically acceptable salt thereof is administered on a regimen of consecutively 2-week treatment and then 1-week interruption. In some embodiments, anlotinib or the pharmaceutically acceptable salt thereof is administered on a regimen of consecutively 5-day treatment and then 2-day interruption. For example, anlotinib or the pharmaceutically acceptable salt thereof can be administered orally at a dose of 6 mg, 8 mg, 10 mg, or 12 mg once daily on a regimen of consecutively 2-week treatment and then 1-week interruption.

Pharmaceutical Composition Comprising Anlotinib or Pharmaceutically Acceptable Salt Thereof

In some embodiments of the present application, the pharmaceutical composition comprising anlotinib or the pharmaceutically acceptable salt thereof comprises 6 mg, 8 mg, 10 mg, or 12 mg of anlotinib or the pharmaceutically acceptable salt thereof. In some embodiments, the pharmaceutical composition is provided in a single dose.

In some embodiments of the present application, according to the administration regimen of 2-week treatment and then 1-week interruption, every 3 weeks is counted as one treatment cycle, and a total dose of anlotinib or the pharmaceutically acceptable salt thereof administered per treatment cycle is 84-168 mg. In some embodiments, a total dose of anlotinib or the pharmaceutically acceptable salt thereof includes an amount selected from 84 mg, 112 mg, 140 mg, 168 mg, or a range formed by any two of the values described above. In some embodiments, a total dose of anlotinib or the pharmaceutically acceptable salt thereof includes 112-168 mg.

In some embodiments, the pharmaceutical composition includes, but is not limited to formulations suitable for oral, parenteral, and local administrations; in some embodiments, the pharmaceutical composition is a formulation suitable for oral administration; in some embodiments, the pharmaceutical composition is a solid formulation suitable for oral administration; in some embodiments, the pharmaceutical composition includes, but is not limited to a tablet and a capsule.

Chemotherapeutic Drug

In some embodiments of the present application, the chemotherapeutic drug includes, but is not limited to one or more of platinum-based anti-tumor drugs (including, but not limited to, oxaliplatin, cisplatin, carboplatin, nedaplatin, dicycloplatin, miriplatin, picoplatin, lobaplatin, triplatin tetranitrate, phenanthriplatin, and satraplatin), camptothecin anti-tumor drugs (including, but not limited to, camptothecin, hydroxycamptothecin, aminocamptothecin, irinotecan, topotecan, exatecan, rubitecan, lurtotecan, gimatecan, karenitecin, and 7-ethylcamptothecin), taxane anti-tumor drugs (including, but not limited to, paclitaxel, paclitaxel liposome, albumin-bound paclitaxel, and docetaxel), nitrogen mustard anti-tumor drugs (including, but not limited to, cyclophosphamide, ifosfamide, chlorambucil, carmustine, melphalan, and bendamustine), antimetabolite anti-tumor drugs (including, but not limited to, fluorouracil anti-tumor drugs (including, but not limited to, carmofur, 5-fluorouracil, tegafur, capecitabine, tegafur-gimeracil-oteracil potassium, difuradin, doxifluridine, and trifluridine), cytosine anti-tumor drugs (including, but not limited to, cytarabine, gemcitabine, azacitidine, and ancitabine), purine anti-tumor drugs (including, but not limited to, mercaptopurine and fludarabine), and antifolate anti-tumor drugs (including, but not limited to, methotrexate and pemetrexed)), anthracycline anti-tumor drugs (including, but not limited to, doxorubicin, epirubicin, pirarubicin, amrubicin, aclarubicin, idarubicin, daunorubicin, mitoxantrone, valrubicin, zorubicin, pixantrone, and liposomal adriamycin), vinblastine anti-tumor drugs (including, but not limited to, vinblastine, vincristine, vindesine, vinflunine, and vinorelbine), and podophyllotoxin anti-tumor drugs (including, but not limited to, etoposide and teniposide), hormone anti-tumor drugs (including, but not limited to, prednisone, prednisolone, dexamethasone, and methylprednisolone sodium succinate), procarbazine, hexamethylmelamine, dacarbazine, mitomycin, actinomycin D (dactinomycin), bleomycin, pingyangmycin, temozolomide, decarbazine, peplomycin, eribulin, plinabulin, sapacitabine, treosulfan, 153Sm-EDTMP, and encequidar.

In some embodiments, the chemotherapeutic drug is selected from the group consisting of one or more of a

platinum-based anti-tumor drug, a taxane anti-tumor drug, and an antimetabolite anti-tumor drug. In some embodiments, the chemotherapeutic drug includes a platinum-based anti-tumor drug, optionally a taxane anti-tumor drug, and optionally an antimetabolite anti-tumor drug. In some embodiments, the chemotherapeutic drug is a platinum-based anti-tumor drug and/or a taxane anti-tumor drug. In some embodiments, the chemotherapeutic drug is a platinum-based anti-tumor drug and/or an antimetabolite anti-tumor drug.

Further, the platinum-based anti-tumor drug is selected from the group consisting of one or more of cisplatin, carboplatin, nedaplatin, dicycloplatin, picoplatin, oxaliplatin, miriplatin, lobaplatin, triplatin tetranitrate, phenanthriplatin, and satraplatin; preferably, the platinum-based anti-tumor drug is carboplatin and/or cisplatin; more preferably, the platinum-based anti-tumor drug is carboplatin. In some embodiments, the taxane anti-tumor drug is selected from the group consisting of one or more of paclitaxel, paclitaxel liposome, albumin-bound paclitaxel, and docetaxel; preferably, the taxane anti-tumor drug is paclitaxel and/or docetaxel and/or paclitaxel liposome; more preferably, the taxane anti-tumor drug is paclitaxel. In some embodiments, the antimetabolite anti-tumor drug is selected from the group consisting of one or more of fluorouracils, cytosines, purines, and antifolates; in some embodiments, the antimetabolite anti-tumor drug is selected from the group consisting of one or more of carmofur, 5-fluorouracil, tegafur, capecitabine, tegafur-gimeracil-oteracil potassium, difuradin, doxifluridine, trifluridine, cytarabine, gemcitabine, azacitidine, ancitabine, mercaptopurine, fludarabine, methotrexate, and pemetrexed; preferably the antimetabolite anti-tumor drug is selected from the group consisting of pemetrexed and/or gemcitabine; more preferably, the antimetabolite anti-tumor drug is pemetrexed. In some embodiments, the chemotherapeutic drug is one or more of carboplatin, pemetrexed, and paclitaxel.

In some embodiments, the chemotherapeutic drug is a platinum-based anti-tumor drug and paclitaxel. In some embodiments, the chemotherapeutic drug is carboplatin and a taxane anti-tumor drug. In some embodiments, the chemotherapeutic drug is carboplatin and paclitaxel. In some embodiments, the chemotherapeutic drug is a platinum-based anti-tumor drug and pemetrexed. In some embodiments, the chemotherapeutic drug is carboplatin and an antimetabolite anti-tumor drug. In some embodiments, the chemotherapeutic drug is carboplatin and pemetrexed.

In some embodiments, the chemotherapeutic drug is administered according to known modes of administration (including doses, routes of administration, and administration regimens).

Anti-PD-L1 Antibody

In some embodiments of the present application, the anti-PD-L1 antibody is one or more of the antibodies disclosed in CN107001463A.

In some embodiments of the present application, the anti-PD-L1 antibody is an isolated anti-PD-L1 antibody; in some embodiments of the present application, the anti-PD-L1 antibody is an anti-PD-L1 humanized monoclonal antibody; in some embodiments of the present application, the anti-PD-L1 antibody is an isolated anti-PD-L1 humanized monoclonal antibody; in some embodiments, the anti-PD-L1 antibody can be an IgG1 or IgG4 antibody; in some embodiments of the present application, the anti-PD-L1 antibody is an IgG1 antibody; in some embodiments, the anti-PD-L1 antibody is a glycosylated IgG1 antibody.

In some embodiments of the present application, the anti-PD-L1 antibody comprises the following amino acid sequences: a heavy chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 1 or SEQ ID NO: 4; a heavy chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 2 or SEQ ID NO: 5; a heavy chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 3 or SEQ ID NO: 6; a light chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 7 or SEQ ID NO: 10; a light chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 8 or SEQ ID NO: 11; and a light chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 9 or SEQ ID NO: 12.

In some embodiments, the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino

acid sequence set forth in SEQ ID NO: 9 or SEQ ID NO: 12.

In some embodiments of the present application, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

In some embodiments of the present application, the isolated anti-PD-L1 antibody described herein comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

Each of the CDR regions described herein and the variants thereof described above are capable of specifically recognizing and binding to PD-L1, thereby effectively blocking the signaling between PD-L1 and PD-1.

In some embodiments of the present application, the anti-PD-L1 antibody comprises the following amino acid sequences: a heavy chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 13 or SEQ ID NO: 14; and a light chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 15 or SEQ ID NO: 16.

In some embodiments of the present application, the anti-PD-L1 antibody comprises the following amino acid sequences: a heavy chain variable region set forth in SEQ ID NO: 13, and a light chain variable region set forth in SEQ ID NO: 15.

In some embodiments of the present application, the anti-PD-L1 antibody comprises the following amino acid sequences: a heavy chain variable region set forth in SEQ ID NO: 14, and a light chain variable region set forth in SEQ ID NO: 16.

In some embodiments of the present application, the anti-PD-L1 antibody comprises the following amino acid sequences: a heavy chain amino acid sequence set forth in SEQ ID NO: 17, and a light chain amino acid sequence set forth in SEQ ID NO: 18.

In some embodiments of the present application, the anti-PD-L1 antibody comprises the following amino acid sequences: a heavy chain amino acid sequence set forth in SEQ ID NO: 19, and a light chain amino acid sequence set forth in SEQ ID NO: 20.

In some embodiments of the present application, the anti-PD-L1 antibody comprises the following amino acid sequences: a heavy chain amino acid sequence set forth in SEQ ID NO: 21, and a light chain amino acid sequence set forth in SEQ ID NO: 18.

In one specific embodiment, the anti-PD-L1 humanized mAb provided herein comprises one or more conservatively substituted variants selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, and SEQ ID NO: 21. The anti-PD-L1 humanized mAb comprising the conservatively substituted variants retains the ability to specifically recognize and bind to PD-L1.

In some embodiments of the present application, the anti-PD-L1 antibody comprises heavy chain complementarity determining regions (CDRs) selected from the group consisting of heavy chain CDRs of antibodies 13C5 and 5G11, and light chain CDRs selected from the group consisting of light chain CDRs of antibodies 13C5 and 5G11. In one embodiment, the anti-PD-L1 antibody described herein comprises: a heavy chain variable region selected from the group consisting of heavy chain variable regions of chimeric antibodies ch5G11-hIgG1, ch5G11-hIgG4, ch13C5-hIgG1, and ch13C5-hIgG4; and a light chain variable region selected from the group consisting of light chain variable regions of chimeric antibodies ch5G 11-hIgG 1, ch5G 11-hIgG4, ch13C5-hIgG1, and ch13C5-hIgG4. In one embodiment, the anti-PD-L1 antibody described herein comprises: a heavy chain variable region selected from the group consisting of heavy chain variable regions of humanized antibodies hu13C5-hIgG1, hu13C5-hIgG4, hu5G11-hIgG1, and hu5G11-hIgG4; and a light chain variable region selected from the group consisting of light chain variable regions of humanized antibodies hu13C5-hIgG1, hu13C5-hIgG4, hu5G11-hIgG1, and hu5G11-hIgG4. Reference can be made to the description of Patent No. WO2016022630 or CN107001463A: 13C5, ch13C5-hIgG1, ch13C5-hIgG4, hu13C5-hIgG1, or hu13C5-hIgG4 comprises an HCDR1 sequence of SYGMS (SEQ ID NO: 4), an HCDR2 sequence of SISSGGSTYYPDSVKG (SEQ ID NO: 5), an HCDR3 sequence of GYDSGFAY (SEQ ID NO: 6), an LCDR1 sequence of ASQSVSTSSSSFMH (SEQ ID NO: 10), an LCDR2 sequence of YASNLES (SEQ ID NO: 11), and an LCDR3 sequence of QHSWEIPYT (SEQ

ID NO: 12); and 5G11, ch5G11-hIgG1, ch5G11-hIgG4, hu5G11-hIgG1, or hu5G11-hIgG4 comprises an HCDR1 sequence of TYGVH (SEQ ID NO: 1), an HCDR2 sequence of VIWRGVTTDYNAAFMS (SEQ ID NO: 2), an HCDR3 sequence of LGFYAMDY (SEQ ID NO: 3), an LCDR1 sequence of KASQSVSNDVA (SEQ ID NO: 7), an LCDR2 sequence of YAANRYT (SEQ ID NO: 8), and an LCDR3 sequence of QQDYTSPYT (SEQ ID NO: 9). In one embodiment, hu5G11-hIgG1 comprises a heavy chain amino acid sequence set forth in SEQ ID NO: 17, and a light chain amino acid sequence set forth in SEQ ID NO: 18.

In some embodiments of the present application, the anti-PD-L1 antibody in the pharmaceutical combination may be selected from the group consisting of one or more antibodies. As used herein, the term "more" refers to more than one, for example, two, three, four, five, or more. For example, in some embodiments of the present application, the anti-PD-L1 antibody is selected from an antibody comprising a heavy chain variable region set forth in SEQ ID NO: 13 and a light chain variable region set forth in SEQ ID NO: 15, or selected from an antibody comprising a heavy chain variable region set forth in SEQ ID NO: 14 and a light chain variable region set forth in SEQ ID NO: 16, or selected from a combination of the aforementioned antibodies. For another example, the anti-PD-L1 antibody is selected from an antibody comprising a heavy chain amino acid sequence set forth in SEQ ID NO: 17 and a light chain amino acid sequence set forth in SEQ ID NO: 18, or selected from an antibody comprising a heavy chain amino acid sequence set forth in SEQ ID NO: 19 and a light chain amino acid sequence set forth in SEQ ID NO: 20, or selected from an antibody comprising a heavy chain amino acid sequence set forth in SEQ ID NO: 21 and a light chain amino acid sequence set forth in SEQ ID NO: 18, or selected from the group consisting of combinations of any two or more of the aforementioned antibodies.

## Pharmaceutical Composition Comprising Anti-PD-L1 Antibody

In some embodiments of the present application, the pharmaceutical composition comprising the anti-PD-L1 antibody comprises 600-2400 mg of the anti-PD-L1 antibody. In some embodiments, the pharmaceutical composition comprises the anti-PD-L1 antibody in an amount selected from the group consisting of 600 mg, 900 mg, 1200 mg, 1500 mg, 1800 mg, 2100 mg, 2400 mg, and a range formed by any two of the values described above. In some embodiments, the pharmaceutical composition comprises 600-2100 mg or 900-1500 mg of the anti-PD-L1 antibody, wherein the pharmaceutical composition comprising the anti-PD-L1 antibody may be present in a multiple-dose or unit-dose form.

In some embodiments of the present application, the pharmaceutical composition comprises 300 mg, 600 mg, or 1200 mg of the anti-PD-L1 antibody. In some embodiments of the present application, provided is a pharmaceutical composition formulated into a unit dose, comprising 300 mg, 600 mg, or 1200 mg of the anti-PD-L1 antibody.

In one specific embodiment, the pharmaceutical composition comprising the anti-PD-L1 antibody is a solution for injection. In some embodiments, the pharmaceutical composition comprising the anti-PD-L1 antibody is an aqueous solution for injection. In some embodiments of the present application, the pharmaceutical composition comprising the anti-PD-L1 antibody comprises one or more of a buffer, an isotonicity modifier, a stabilizer, and/or a surfactant. In particular, the pharmaceutical composition comprising the anti-PD-L1 antibody comprises an anti-PD-L1 antibody (e.g., mAb) at 1-150 mg/mL, a buffer at 3-50 mM, an isotonicity modifier/stabilizer at 2-150 mg/mL, and a surfactant at 0.01-0.8 mg/mL, and has a pH of 4.5-6.8.

In some embodiments of the present application, in the pharmaceutical composition comprising the anti-PD-L1 antibody, the anti-PD-L1 mAb is at a concentration of 5-150 mg/mL (w/v); in some embodiments, the concentration is 10-60 mg/mL (w/v); in some embodiments, the concentration is 10-30 mg/mL (w/v). In some specific embodiments, the anti-PD-L1 mAb is at a concentration of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, or 120 mg/mL (w/v); in some embodiments, the concentration is 10 mg/mL, 20 mg/mL, 30 mg/mL, 40 mg/mL, 50 mg/mL, or 60 mg/mL (w/v); in some embodiments, the concentration is 10 mg/mL, 20 mg/mL, or 30 mg/mL (w/v). In some embodiments, the anti-PD-L1 mAb is at a concentration of 10 mg/mL (w/v). In other embodiments, the anti-PD-L1 mAb is at a concentration of 30 mg/mL (w/v). In other embodiments, the anti-PD-L1 mAb is at a concentration of 60 mg/mL (w/v).

In some embodiments of the present application, the buffer is a histidine salt buffer. The histidine salt buffer is at a concentration of 5-30 mM; in some embodiments, the concentration is 10-25 mM; in some embodiments, the concentration is 10-20 mM; in some embodiments, the concentration is 10-15 mM. In some specific embodiments, the histidine salt buffer is at a concentration of 5 mM, 10 mM, 15 mM, 20 mM, 25 mM, or 30 mM. In some embodiments, the histidine salt buffer is at a concentration of 10 mM. In other embodiments, the histidine salt buffer is at a concentration of 15 mM. In other embodiments, the histidine salt buffer is at a concentration of 20 mM. The histidine salt buffer comprises histidine and hydrochloric acid.

In some embodiments of the present application, the isotonicity modifier/stabilizer is sucrose at 20-150 mg/mL (w/v); in some embodiments, the isotonicity modifier/stabilizer is sucrose at 40-100 mg/mL (w/v); in some embodiments, the isotonicity modifier/stabilizer is sucrose at 60-80 mg/mL (w/v). In some specific embodiments, the sucrose is at a concentration of 40 mg/mL, 50 mg/mL, 60 mg/mL, 70 mg/mL, 80 mg/mL, 90 mg/mL, or 100 mg/mL. In some specific

embodiments, the sucrose is at a concentration of 60 mg/mL. In some specific embodiments, the sucrose is at a concentration of 70 mg/mL. In some specific embodiments, the sucrose is at a concentration of 80 mg/mL. In some specific embodiments, the sucrose is at a concentration of 90 mg/mL.

In some embodiments of the present application, the surfactant is selected from the group consisting of polysorbate 80, polysorbate 20, and poloxamer 188; in some embodiments, the surfactant is selected from the group consisting of polysorbate 80 and polysorbate 20; in some embodiments, the surfactant is selected from polysorbate 80. In some embodiments, the surfactant is at a concentration of 0.05-0.6 mg/mL (w/v); in some embodiments, the concentration is 0.1-0.4 mg/mL (w/v); in some embodiments, the concentration is 0.2-0.3 mg/mL (w/v).

In some embodiments of the present application, the surfactant is polysorbate 80 or polysorbate 20 at 0.01-0.8 mg/mL (w/v). In some specific embodiments, the surfactant is polysorbate 80 at 0.05-0.6 mg/mL; in some embodiments, the surfactant is polysorbate 80 at 0.1-0.4 mg/mL; in some embodiments, the surfactant is polysorbate 80 at 0.2-0.3 mg/mL; in some embodiments, the surfactant is polysorbate 80 at 0.2 mg/mL. In some embodiments, in the pharmaceutical composition, polysorbate 80 is at a content of 0.1 mg/mL, 0.2 mg/mL, 0.3 mg/mL, 0.4 mg/mL, 0.5 mg/mL, or 0.6 mg/mL; in some embodiments, in the pharmaceutical composition, polysorbate 80 is at a content of 0.2 mg/mL, 0.3 mg/mL, 0.4 mg/mL, or 0.5 mg/mL; in some embodiments, in the pharmaceutical composition, polysorbate 80 is at a content of 0.2 mg/mL, 0.3 mg/mL, or 0.4 mg/mL; in some embodiments, in the pharmaceutical composition, polysorbate 80 is at a content of 0.2 mg/mL. In some embodiments, in the pharmaceutical composition, polysorbate 80 is at a content of 0.1 mg/mL. In other embodiments, in the pharmaceutical composition, polysorbate 80 is at a content of 0.2 mg/mL. In some embodiments, in the pharmaceutical composition, polysorbate 80 is at a content of 0.3 mg/mL. In other embodiments, in the pharmaceutical composition, polysorbate 80 is at a content of 0.4 mg/mL. In some embodiments, in the pharmaceutical composition, polysorbate 80 is at a content of 0.5 mg/mL.

In some embodiments of the present application, the aqueous solution of the pharmaceutical composition has a pH selected from 4.0-6.8; in some embodiments, the pH is 4.5-6.5; in some embodiments, the pH is 5.5-6.0; in some embodiments, the pH is 5.5. In some embodiments, the aqueous solution of the pharmaceutical composition has a pH of 4.5, 4.8, 5.0, 5.2, 5.4, 5.5, 5.6, 5.8, or 6.0; in some embodiments, the pH is 5.0, 5.2, 5.4, 5.5, or 5.6; in some embodiments, the pH is 5.5. In some embodiments, the aqueous solution of the pharmaceutical composition has a pH of 5.0. In some embodiments, the aqueous solution of the pharmaceutical composition has a pH of 5.2. In some embodiments, the aqueous solution of the pharmaceutical composition has a pH of 5.4. In some embodiments, the aqueous solution of the pharmaceutical composition has a pH of 5.5. In some embodiments, the aqueous solution of the pharmaceutical composition has a pH of 5.6. In some embodiments, the aqueous solution of the pharmaceutical composition has a pH of 5.8. In some embodiments, the aqueous solution of the pharmaceutical composition has a pH of 6.0.

In some specific embodiments of the present application, the pharmaceutical composition comprises: (a) an anti-PD-L1 antibody at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally hydrochloric acid of a suitable amount for adjusting the pH of the composition to 5.0. In one specific embodiment of the present application, the pharmaceutical composition comprises: (a) an anti-PD-L1 mAb at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally hydrochloric acid of a suitable amount for adjusting the pH of the composition to 5.0.

In another specific embodiment of the present application, the pharmaceutical composition comprises: (a) an anti-PD-L1 antibody at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) an anti-PD-L1 antibody at a concentration of 50 mg/mL (w/v), (b) sucrose at a concentration of 80 mg/mL (w/v), (c) polysorbate 80 at a concentration of 0.3 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) an anti-PD-L1 antibody at a concentration of 100 mg/mL (w/v), (b) sucrose at a concentration of 80 mg/mL (w/v), (c) polysorbate 80 at a concentration of 0.5 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) an anti-PD-L1 antibody at a concentration of 30 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) an anti-PD-L1 antibody at a concentration of 60 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) an anti-PD-L1 antibody at a concentration of 10 mg/mL (w/v), (b) sucrose at a concentration of 70 mg/mL (w/v), (c) polysorbate 80 at a concentration of 0.4 mg/mL (w/v), (d) histidine at a concentration of 20 mM, and (e) optionally acetic acid of a suitable amount for adjusting the pH of the composition to 6.5.
In another specific embodiment of the present application, the pharmaceutical composition comprises: (a) an anti-PD-L1 mAb at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 20 mM, and (e) optionally hydrochloric acid of a suitable amount for adjusting the pH of the composition to 5.5.
In another specific embodiment of the present application, the pharmaceutical composition is a water-soluble injection; in some embodiments, the water-soluble injection includes, but is not limited to, a water-soluble formulation without lyophilization or a water-soluble formulation reconstituted from a lyophilized powder. In other embodiments, the pharmaceutical composition is a lyophilized formulation. The lyophilized formulation refers to a formulation prepared by subjecting an aqueous solution to a lyophilization process in which the substance is first frozen, and then the amount of the solvent is reduced by sublimation (primary drying process) and then by desorption (secondary drying process) until the amount of the solvent is reduced to a value that no longer supports the biological activity or a chemical reaction. The lyophilized formulation of the present application can also be dried by other methods known in the art, such as spray drying and bubble drying.

Non-Small Cell Lung Cancer

In some embodiments of the present application, the non-small cell lung cancer is non-small cell lung cancer that is inoperable, and/or not amenable to curative concurrent radiotherapy and chemotherapy, and/or advanced, and/or recurrent, and/or metastatic; in some embodiments, the non-small cell lung cancer is locally advanced (stage IIIB/IIIC) non-small cell lung cancer; in some embodiments, the non-small cell lung cancer is locally advanced (stage IIIB/IIIC) non-small cell lung cancer that is inoperable and not amenable to curative concurrent radiotherapy and chemotherapy; in some embodiments, the non-small cell lung cancer is metastatic non-small cell lung cancer that is inoperable and not amenable to curative concurrent radiotherapy and chemotherapy; the non-small cell lung cancer is recurrent (stage IV) non-small cell lung cancer that is inoperable and not amenable to curative concurrent radiotherapy and chemotherapy; in some embodiments, the non-small cell lung cancer is refractory non-small cell lung cancer. In some embodiments, the metastatic non-small cell lung cancer includes, but is not limited to, single metastasis, disseminated metastasis, and diffuse metastasis of a lesion; the metastatic lesions include, but are not limited to, lymph nodes, pleura, bone, brain, pericardium, adrenal gland, and liver; in some embodiments, the non-small cell lung cancer is non-small cell lung cancer with brain metastasis.
In some embodiments of the present application, the non-small cell lung cancer is driver-negative non-small cell lung cancer; in some embodiments of the present application, the non-small cell lung cancer is EGFR and/or ALK wild-type non-small cell lung cancer. In some embodiments of the present application, the non-small cell lung cancer is EGFR and ALK wild-type non-small cell lung cancer. In some embodiments of the present application, the non-small cell lung cancer is non-small cell lung cancer that is driver-negative, and/or inoperable, and/or locally advanced, and/or metastatic.
In some embodiments of the present application, the non-small cell lung cancer includes, but is not limited to, adenocarcinoma, squamous cell carcinoma, adenosquamous carcinoma, large cell carcinoma, sarcoid carcinoma, and combined lung cancer. In some embodiments, the non-small cell lung cancer is squamous non-small cell lung cancer. In some embodiments, the non-small cell lung cancer is lung adenocarcinoma. In some embodiments, the combined lung cancer is non-small cell lung cancer that does not include small cell lung cancer.
In some embodiments, the non-small cell lung cancer is squamous non-small cell lung cancer without an EGFR-sensitive mutation and or ALK fusion. In some embodiments, the non-small cell lung cancer is non-squamous non-small cell lung cancer. In some embodiments, the non-squamous non-small cell lung cancer is non-small cell lung cancer other than squamous cell carcinoma. In some embodiments, the non-small cell lung cancer is non-squamous non-small cell lung cancer without an EGFR-sensitive mutation or an ALK fusion.
In some embodiments of the present application, the patient with the non-small cell lung cancer has not previously received any treatment; in some embodiments, the patient with the non-small cell lung cancer has not previously received systemic anti-tumor treatment; in some embodiments, the patient with the non-small cell lung cancer has not previously received treatment with a chemotherapy regimen; in some embodiments, the patient with the non-small cell lung cancer has not received systemic anti-tumor treatment for advanced, and/or recurrent, and/or metastatic non-small cell lung cancer; in some embodiments, the patient with the non-small cell lung cancer has previously received neoadjuvant and/or adjuvant chemotherapy.
In some embodiments, the patient with the non-small cell lung cancer has previously received treatment with at least one chemotherapy regimen; in some embodiments, the patient with the non-small cell lung cancer has previously

experienced disease recurrence following treatment with at least one chemotherapy regimen; in some embodiments, the patient with the non-small cell lung cancer has previously received treatment with at least two chemotherapy regimens; in some embodiments, the patient with the non-small cell lung cancer has previously experienced disease recurrence following adjuvant chemotherapy; in some embodiments, the patient with the non-small cell lung cancer has previously received adjuvant chemotherapy, with time interval between the last adjuvant chemotherapy and the disease recurrence being at least 6 months. In some embodiments, the patient with the non-small cell lung cancer has previously received breast-directed radiotherapy, with time interval between the end of the radiotherapy and the current treatment being at least 6 months. In some embodiments, the patient with the non-small cell lung cancer has previously received palliative radiotherapy directed to parts other than the breast, with time interval between the radiotherapy and the current treatment being more than 7 days.

Mode of Administration

The content below is not intended to limit the mode of administration of the pharmaceutical combination of the present application.

The components in the pharmaceutical combination of the present application can each be independently administered, or some or all of the components are co-administered, by various proper routes, including, but not limited to, oral administration or parenteral administration (by intravenous, intramuscular, topical, or subcutaneous routes). In some embodiments, the components in the pharmaceutical combination of the present application can each be independently administered, or some or all of the components are co-administered, orally or by injection, for example, intravenous injection or intraperitoneal injection.

The components in the pharmaceutical combination of the present application can each be independently suitable dosage forms, or some or all of the components are co-formulated in a suitable dosage form, including, but not limited to, a tablet, a lozenge, a pill, a capsule (for example, hard capsule, soft capsule, enteric capsule, or microcapsule), an elixir, a syrup, an injection (intramuscular, intravenous or intraperitoneal injection), a granule, an emulsion, a suspension, a solution, a dispersant, and dosage forms of sustained-release formulations for oral or non-oral administration.

The components in the pharmaceutical combination of the present application can each independently contain a pharmaceutically acceptable carrier and/or excipient, or some or all of the components are co-formulated with a pharmaceutically acceptable carrier and/or excipient.

Administration Regimen

In some embodiments of the present application, in the use or the method described above, the anti-PD-L1 antibody and at least one chemotherapeutic drug, and optionally, anlotinib or the pharmaceutically acceptable salt thereof, are each present in the form of a pharmaceutical composition and can be administered simultaneously, sequentially or intermittently.

In some embodiments of the present application, in the use or the method described above, the anti-PD-L1 antibody and at least one chemotherapeutic drug, and optionally, anlotinib or the pharmaceutically acceptable salt thereof, are each administered intermittently. In some embodiments, the anti-PD-L1 antibody and at least one chemotherapeutic drug, and optionally, anlotinib or the pharmaceutically acceptable salt thereof, are each administered according to the same or different administration regimens. In some embodiments, the anti-PD-L1 antibody and at least one chemotherapeutic drug, and optionally, anlotinib or the pharmaceutically acceptable salt thereof, are each administered according to different administration regimens.

In some embodiments of the present application, in the use or the method described above, the anti-PD-L1 antibody can be administered once every week (q1w), once every 2 weeks (q2w), once every 3 weeks (q3w), or once every 4 weeks (q4w). In one specific embodiment, the anti-PD-L1 antibody is administered once every 3 weeks. In some embodiments, the anti-PD-L1 antibody is administered at a dose of 600-2400 mg each time.

In some embodiments of the present application, anlotinib or the pharmaceutically acceptable salt thereof can be administered at a dose of 6 mg, 8 mg, 10 mg or 12 mg once daily on an administration regimen of consecutively 2-week treatment and then 1-week interruption. In this case, every 3 weeks is counted as one treatment cycle.

In some embodiments of the present application, the chemotherapeutic drug can be administered according to known modes of administration.

In some embodiments of the present application, the anti-PD-L1 antibody and at least one chemotherapeutic drug, and optionally, anlotinib or the pharmaceutically acceptable salt thereof, each have the same or different treatment cycles. In some specific embodiments, the anti-PD-L1 antibody and at least one chemotherapeutic drug, and optionally, anlotinib or a pharmaceutically acceptable salt thereof, have the same treatment cycle, e.g., with every 1 week, every 2 weeks, every 3 weeks, or every 4 weeks counted as one treatment cycle. In some embodiments, every three weeks is counted as one treatment cycle.

In some embodiments of the present application, the administration regimen of the pharmaceutical combination of the present application or the kit of the present application comprises a first treatment phase and optionally a second treatment phase. It can be understood that the second treatment phase is performed sequentially after the first treatment phase.

The administration regimen of the present application is also suitable for the use for preparing a medicament for use in treating non-small cell lung cancer, a method for treating non-small cell lung cancer in a patient, and the use for treating non-small cell lung cancer in a patient.

In some embodiments of the present application, the first treatment phase comprises 1-10 treatment cycles, preferably 2-8 treatment cycles, further preferably 4-6 treatment cycles, and most preferably 4 treatment cycles.

In some embodiments of the present application, the second treatment phase comprises 2-20 treatment cycles. In some embodiments, the second treatment phase was carried out until loss of clinical benefits, intolerable toxicity, PD upon efficacy assessment, or investigator-assessed ineligibility for further treatment.

In some embodiments of the present application, the treatment cycle comprises 14-42 days; in some embodiments, the treatment cycle comprises 14 days, 21 days, 28 days, 35 days, or 42 days; in some embodiments, the treatment cycle comprises 21 days. In some specific embodiments, the first treatment phase has the same treatment cycle as the second treatment phase. In some specific embodiments, the first treatment phase and the second treatment phase both have a treatment cycle of 21 days.

In some embodiments of the present application, the anti-PD-L1 antibody is administered once on day 1, 2, 3, 4, 5, 6, or 7 of each treatment cycle, preferably once on day 1 of each treatment cycle.

In some embodiments of the present application, anlotinib or the pharmaceutically acceptable salt thereof is administered continuously on days 1-7, days 7-14, days 1-14, or days 7-21 of each treatment cycle, preferably on days 1-14 of each treatment cycle.

In some embodiments, the chemotherapeutic drug is administered according to known modes of administration (including doses, routes of administration, and administration regimens).

The present application provides a method for treating squamous non-small cell lung cancer, comprising administering to a patient in need thereof a first pharmaceutical combination in a first treatment phase; and optionally, administering to the patient in need thereof a second pharmaceutical combination in a second treatment phase. The present application further provides use of the pharmaceutical combination described above for preparing a medicament for use in treating squamous non-small cell lung cancer in a patient, and use of the pharmaceutical combination described above for treating squamous non-small cell lung cancer in a patient. The pharmaceutical combination comprises a first pharmaceutical combination administered to a patient in need thereof in a first treatment phase, and optionally a second pharmaceutical combination administered to the patient in need thereof in a second treatment phase.

In some embodiments, the administration regimen for the first treatment phase comprises: 1) administering the anti-PD-L1 antibody once on one of days 1-7 of each treatment cycle; and optionally; 2) administering the taxane anti-tumor drug once on one of days 1-7 of each treatment cycle; and optionally, 3) administering the platinum-based anti-tumor drug once on one of days 1-7 of each treatment cycle.

In some embodiments of the present application, the administration regimen for the first treatment phase comprises: 1) administering the anti-PD-L1 antibody once on day 1 of each treatment cycle; and optionally, 2) administering the taxane anti-tumor drug once on day 1 of each treatment cycle; and optionally, 3) administering a platinum-based anti-tumor drug once on day 1 of each treatment cycle.

In some embodiments of the present application, the administration regimen for the first treatment phase comprises: 1) administering the anti-PD-L1 antibody once on day 1 of each treatment cycle; and optionally, 2) administering paclitaxel once on day 1 of each treatment cycle; and optionally, 3) administering carboplatin once on day 1 of each treatment cycle.

In some embodiments, the administration regimen for the first treatment phase comprises: administering the anti-PD-L1 antibody, paclitaxel, and carboplatin sequentially on day 1 of each treatment cycle.

In some embodiments of the present application, the administration regimen for the second treatment phase comprises: 1) administering the anti-PD-L1 antibody once on one of days 1-7 of each treatment cycle; and 2) administering anlotinib or the pharmaceutically acceptable salt thereof continuously on days 1-28 of each treatment cycle.

In some embodiments of the present application, the administration regimen for the second treatment phase comprises: 1) administering the anti-PD-L1 antibody once on day 1 of each treatment cycle; and 2) administering anlotinib or the pharmaceutically acceptable salt thereof continuously on days 1-14 of each treatment cycle.

The present application provides a method for treating non-squamous non-small cell lung cancer or lung adenocarcinoma, comprising administering to a patient in need thereof a first pharmaceutical combination in a first treatment phase; and optionally, administering to the patient in need thereof a second pharmaceutical combination in a second treatment phase. The present application further provides use of the pharmaceutical combination described above for preparing a medicament for treating non-squamous non-small cell lung cancer or lung adenocarcinoma in a patient and use of the pharmaceutical combination described above for treating non-squamous non-small cell lung cancer or lung

adenocarcinoma in a patient. The pharmaceutical combination comprises a first pharmaceutical combination administered to a patient in need thereof in a first treatment phase, and optionally a second pharmaceutical combination administered to the patient in need thereof in a second treatment phase.

In some embodiments, the administration regimen for the first treatment phase comprises: 1) administering the anti-PD-L1 antibody once on one of days 1-7 of each treatment cycle; and optionally, 2) administering the antimetabolite anti-tumor drug once on one of days 1-7 of each treatment cycle; and optionally, 3) administering the platinum-based anti-tumor drug once on one of days 1-7 of each treatment cycle.

In some embodiments of the present application, the administration regimen for the first treatment phase comprises: 1) administering the anti-PD-L1 antibody once on day 1 of each treatment cycle; and optionally, 2) administering the antimetabolite anti-tumor drug once on day 1 of each treatment cycle; and optionally, 3) administering the platinum-based anti-tumor drug once on day 1 of each treatment cycle.

In some embodiments of the present application, the administration regimen for the first treatment phase comprises: 1) administering the anti-PD-L1 antibody once on day 1 of each treatment cycle; and optionally, 2) administering pemetrexed once on day 1 of each treatment cycle; and optionally, 3) administering carboplatin once on day 1 of each treatment cycle.

In some embodiments, the administration regimen for the first treatment phase comprises: administering the anti-PD-L1 antibody, pemetrexed, and carboplatin sequentially on day 1 of each treatment cycle.

In some embodiments of the present application, the administration regimen for the second treatment phase comprises: 1) administering the anti-PD-L1 antibody once on one of days 1-7 of each treatment cycle; and 2) administering anlotinib or the pharmaceutically acceptable salt thereof continuously on days 1-28 of each treatment cycle; and 3) administering pemetrexed once on one of days 1-7 of each treatment cycle.

In some embodiments of the present application, the administration regimen for the second treatment phase comprises: 1) administering the anti-PD-L1 antibody once on day 1 of each treatment cycle; and 2) administering anlotinib or the pharmaceutically acceptable salt thereof continuously on days 1-14 of each treatment cycle; and 3) administering pemetrexed once on day 1 of each treatment cycle.

## Technical Effects

In some embodiments, the pharmaceutical combination of the present application can safely and effectively treat non-small cell lung cancer, particularly non-small cell lung cancer that is inoperable, and/or not amenable to curative concurrent radiotherapy and chemotherapy, and/or advanced, and/or recurrent, and/or metastatic. In some embodiments, the pharmaceutical combination of the present application can safely and effectively treat EGFR and/or ALK wild-type non-small cell lung cancer. In some embodiments, the pharmaceutical combination of the present application can safely and effectively treat squamous non-small cell lung cancer, particularly squamous non-small cell lung cancer that is inoperable, and/or not amenable to curative concurrent radiotherapy and chemotherapy, and/or advanced, and/or recurrent, and/or metastatic. In some embodiments, the pharmaceutical combination of the present application can safely and effectively treat non-squamous non-small cell lung cancer or lung adenocarcinoma, particularly non-squamous non-small cell lung cancer or lung adenocarcinoma that is inoperable, and/or not amenable to curative concurrent radiotherapy and chemotherapy, and/or advanced, and/or recurrent, and/or metastatic.

In some embodiments, the pharmaceutical combination of the present application can provide a treatment with higher tolerability in patients, and as compared with the administration of any one or two drugs of the combination, has a better anti-tumor effect and/or fewer adverse effects and/or complications. In some embodiments, the pharmaceutical combination of the present application can exhibit a superior synergistic anti-tumor effect in the treatment of non-small cell lung cancer.

In some embodiments, the patient with the non-small cell lung cancer has a significantly prolonged median PFS (progression-free survival) after receiving treatment with the pharmaceutical combination of the present application. In some embodiments, after receiving the treatment, the median PFS of the patient is up to or more than 6.5 months, up to or more than 7 months, up to or more than 7.5 months, up to or more than 8 months, up to or more than 9 months, up to or more than 10 months, up to or more than 11 months, up to or more than 12 months, up to or more than 13 months, up to or more than 14 months, or up to or more than 15 months.

In some embodiments, the patient with the non-small cell lung cancer has a significantly prolonged ORR in the patient after receiving treatment with the pharmaceutical combination of the present application. In some embodiments, after receiving the treatment, the ORR of the patient is up to or more than 30%, up to or more than 35%, up to or more than 40%, up to or more than 50%, up to or more than 60%, up to or more than 70%, or up to or more than 80%.

## Definitions and Explanations

Unless otherwise stated, the following terms used in the present application shall have the following meanings.

A certain term, unless otherwise specifically defined, should not be considered uncertain or unclear, but construed according to its common meaning in the art. When referring to a trade name, it is intended to refer to its corresponding commercial product or its active ingredient.

Herein, unless otherwise stated, the amount of anlotinib or the pharmaceutically acceptable salt thereof mentioned herein refers to the amount of the active ingredient anlotinib free base.

Unless otherwise stated, the term "dose" refers to a dose administered to a patient without considering the weight or the body surface area (BSA) of the patient. For example, a 60 kg human and a 100 kg human will receive the same dose of antibody (e.g., 240 mg of anti-PD-1 antibody).

Herein, "complete response" (CR) refers to disappearance of all target lesions.

Herein, "partial response" (PR) refers to a reduction in the total diameter of the target lesion by 30% or more compared to the baseline total diameter.

Herein, "progressive disease" (PD) refers to an increase in the total diameter of the target lesion by 20% or more compared to the minimum value of the total diameter found in the study, including the baseline total diameter if it is the minimum value in the study. Besides the relative increase of 20 % or more, the absolute value of the total diameter must be increased by 5 mm or more. The appearance of one or more new lesions should also be considered as progressive disease.

Herein, "stable disease" (SD) refers to the shrinkage of the lesion, but not enough for the evaluation criteria for PR, or the enlargement of the lesion, but not enough for the evaluation criteria for PD, with the minimum diameter used as a reference in the trial.

Herein, "best response" refers to the best therapeutic effect at all time points from initial evaluation, by the end of the last evaluation.

As used herein, the term "pharmaceutical combination" refers to a combination of two or more active ingredients (administered as the respective active ingredients themselves, or as their respective derivatives such as pharmaceutically acceptable salts or esters, prodrugs, or compositions) that are administered simultaneously or successively. The active substances can be administered to a subject simultaneously or sequentially in any order as a single formulation.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include a Fab fragment, a Fab' fragment, an $F(ab')_2$ fragment, an Fv fragment, an isolated CDR region, a single chain Fv molecule (scFv), an Fd fragment, and other antibody fragments known in the art. The antibody and the fragment thereof may also include a recombinant polypeptide, a fusion protein, and a bispecific antibody. The anti-PD-L1 antibody and the fragment thereof described herein can be of IgG1, IgG2, IgG3, or IgG4 isotype. The term "isotype" refers to the class of antibodies encoded by the heavy chain constant region gene. In one embodiment, the anti-PD-L1 antibody and the fragment thereof described herein are of the IgG1 or IgG4 isotype. The anti-PD-L1 antibody and the fragment thereof of the present application can be derived from any species including, but not limited to, mouse, rat, rabbit, primate, llama, and human. The anti-PD-L1 antibody and the fragment thereof can be a chimeric antibody, a humanized antibody, or an intact human antibody. In one embodiment, the anti-PD-L1 antibody is an antibody produced by a hybridoma cell line derived from a mouse. Thus, in one embodiment, the anti-PD-L1 antibody is a murine antibody. In another embodiment, the anti-PD-L1 antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The term "humanized antibody" refers to an antibody comprising complementarity determining regions (CDRs) derived from a non-human antibody, and framework and constant regions derived from a human antibody. For example, an anti-PD-L1 antibody described herein may comprise CDRs derived from one or more murine antibodies as well as human framework and constant regions. Thus, in one embodiment, the humanized antibody described herein binds to the same epitope on PD-L1 as the murine antibody from which the CDRs of the humanized antibody are derived. In some embodiments, the anti-PD-L1 antibody is a humanized antibody. Additional anti-PD-L1 antibodies or variants thereof comprising the heavy and light chain CDRs provided herein can be generated using any human framework sequences, and are also included in the present application. In one embodiment, framework sequences suitable for use in the present application include those similar in structure to the framework sequences provided herein. Additional modifications may be made in the framework regions to improve the properties of the antibodies provided herein. Such additional framework modifications may include: chemical modifications, point mutations for reducing immunogenicity or removing T cell epitopes, or back mutations to residues in original germline sequences. In some embodiments, such modifications include those corresponding to the mutations exemplified herein, including back mutations to germline sequences. For example, in one embodiment, one or more amino acids in the human VH and/or VL framework regions of the humanized antibodies described herein are back-mutated to the corresponding amino acids in the parent murine antibodies. For example, for the VH and VL of humanized 5G11 and humanized 13C5 antibodies, several sites of

framework amino acids of the template human antibodies described above are back-mutated to the corresponding amino acid sequences in the mouse 5G11 and 13C5 antibodies. In one embodiment, the amino acids at positions 53, 60, and/or 67 of the light chain variable region are back-mutated to the corresponding amino acids found at the positions in mouse 5G11 or 13C5 light chain variable region. In another embodiment, the amino acids at positions 24, 28, 30, 49, 73, 83, and/or 94 of the heavy chain variable region are back-mutated to the corresponding amino acids found at the positions in mouse 5G11 or 13C5 heavy chain variable region. In one embodiment, the humanized 5G11 antibody comprises: a light chain variable region, wherein the amino acid at position 60 is mutated from Ser (S) to Asp (D) and the amino acid at position 67 is mutated from Ser (S) to Tyr (Y); and a heavy chain variable region, wherein the amino acid at position 24 is mutated from Phe (F) to Val (V), the amino acid at position 49 is mutated from Ala (A) to Gly (G), the amino acid at position 73 is mutated from Thr (T) to Asn (N), and the amino acid at position 83 is mutated from Thr (T) to Asn (N). In one embodiment, the humanized 13C5 antibody comprises: a light chain variable region, wherein the amino acid at position 53 is mutated from Tyr (Y) to Lys (K); and a heavy chain variable region, wherein the amino acid at position 28 is mutated from Thr (T) to Ile (I), the amino acid at position 30 is mutated from Ser (S) to Arg (R), the amino acid at position 49 is mutated from Ser (S) to Ala (A), and the amino acid at position 94 is mutated from Tyr (Y) to Asp (D). Additional or alternative back mutations can be made in the framework regions of the humanized antibodies provided herein to improve the properties of the antibodies. The present application further includes humanized antibodies that bind to PD-L1 and comprise framework modifications corresponding to the exemplary modifications described herein relative to any suitable framework sequence, as well as other framework modifications that otherwise improve antibody properties.

For the isolated antibody or the fragment thereof described herein that binds to PD-L1, the antibody can be produced by a hybridoma selected from the group consisting of the hybridomas designated herein as 13C5 and 5G11. Accordingly, the antibody described herein further includes hybridomas 13C5 and 5G11, and any hybridomas that produce the antibodies disclosed herein. The present application further comprises an isolated polynucleotide encoding the antibody and the fragment thereof provided herein. The present application further comprises an expression vector comprising the isolated polynucleotide, and a host cell comprising the expression vector.

The "isolated antibody" refers to an antibody that is substantially free of other antibodies having different antigenic specificities (e.g., an isolated antibody that specifically binds to PD-1 is substantially free of antibodies that specifically bind to antigens apart from PD-1). However, an isolated antibody that specifically binds to PD-1 may have cross-reactivity with other antigens (such as PD-1 molecules from different species). Furthermore, the isolated antibody may be substantially free of other cellular materials and/or chemicals.

The term "monoclonal antibody" ("mAb") refers to a non-naturally occurring preparation of antibody molecules of an individual molecule component (i.e., antibody molecules whose base sequences are substantially identical and which exhibit a single binding specificity and affinity for a particular epitope). mAb is an example of the isolated antibody. mAbs can be produced by hybridoma techniques, recombinant techniques, transgenic techniques, or other techniques known to those skilled in the art.

The antibody or the antigen-binding fragment thereof described herein is specific for PD-L1. In one embodiment, the antibody or/and the fragment thereof is specific for PD-L1. In one embodiment, the antibody or the fragment thereof described herein binds to human or primate PD-L1, but does not bind to PD-L1 from any other mammals. In another embodiment, the antibody or/and the fragment thereof does not bind to mouse PD-L1. The terms "human PD-L1", "hPD-L1", "huPD-L1", and the like are used interchangeably herein and refer to human PD-L1 and variants or isotypes of human PD-L1. The terms "specific", "specificity" and "specifically" mean that the antibody and the fragment thereof bind to PD-L1 with greater affinity than any other targets.

The term "treat", "treating", or "treatment" usually refers to acquiring needed pharmacological effects and/or physiological effects. In terms of partially or fully stabilizing or curing a disease and/or an adverse effect of the disease, the effect can be therapeutic. As used herein, "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "effective amount" refers to an amount of the compound of the present application for (i) treating a specific disease, condition or disorder, (ii) alleviating, ameliorating or eliminating one or more symptoms of a specific disease, condition or disorder, or (iii) preventing or delaying onset of one or more symptoms of the specific disease, condition or disorder described herein. The amount of active substance (e.g., the antibody or compound of the present application) constituting the "therapeutically effective amount" may vary according to factors such as the disease state, age, sex, and weight of the individual, and the ability of a therapeutic agent or a combination of therapeutic agents to elicit a desired response in the individual. The effective amount may also be determined routinely by those skilled in the art in accordance with their knowledge and the present disclosure. The terms "administer", "administration", or "administering" refers to physically introducing the composition comprising a therapeutic agent to an entity using any of a variety of methods and delivery systems known to those skilled in the art. Routes of administration of immune checkpoint inhibitors (e.g., an anti-PD-1 antibody or an anti-PD-L1 antibody) include parenteral routes of administration (including

but not limited to intravenous, intramuscular, subcutaneous, intraperitoneal, spinal, or other parenteral routes of administration), for example, by injection or infusion. In some embodiments, the phrase "parenteral administration" and "administered parenterally" as used herein are used interchangeably, and typically refer to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In some embodiments, the immune checkpoint inhibitor (e.g., an anti-PD-1 antibody or an anti-PD-L1 antibody) is administered by a non-parenteral route, and in some embodiments, by oral administration; other non-parenteral routes include local, epidermal or mucosal administration route, e.g., intranasal, intravaginal, intrarectal, sublingual or local route of administration. The administration may also be performed, e.g., once, multiple times, and/or over one or more extended periods of time.

The term "pharmaceutically acceptable" is used herein for those compounds, materials, compositions, and/or dosage forms which are suitable for use in contact with the tissues of human beings and animals without excessive toxicity, irritation, allergic response, or other problems or complications.

The term "pharmaceutically acceptable salt" includes salts formed from a free base and an acid and salts formed from an acid and a free base, for example, hydrochloride, hydrobromide, nitrate, sulfate, phosphate, formate, acetate, trifluoroacetate, fumarate, oxalate, maleate, citrate, succinate, mesylate, benzenesulfonate and p-methylbenzenesulfonate; in some embodiments, the pharmaceutically acceptable salt is hydrochloride, hydrobromide, sulfate, formate, acetate, trifluoroacetate, fumarate, maleate, mesylate, p-methylbenzenesulfonate, sodium salt, potassium salt, ammonium salt and amino acid salt, or the like. In the present application, in forming a pharmaceutically acceptable salt, the acid and the free base are in a molar ratio of 1:0.2-1:5; in some embodiments, the molar ratio is 1:0.5, 1:1, 1:2, 1:3, 1:4, 1:5, 1:6, 1:7, or 1:8.

Herein, the "patient" is a mammal, and in some embodiments, the patient is a human. In some embodiments, the subject is a human. "Entity" includes any human or non-human animal. The terms "patient", "entity", and "subject" are used interchangeably herein in certain contexts.

The term "chemotherapy regimen" includes one or more chemotherapeutic drugs.

The term "unit dose" refers to the smallest unit of packaging containing a certain quantity of pharmaceutical product; for example, in a box of seven capsules, each capsule is a unit dose; or a vial of injection is a unit dose. The term "multiple dose" consists of multiple unit doses.

The term "pharmaceutical composition" refers to a mixture consisting of one or more of the active ingredients or pharmaceutical combinations thereof of the present application and a pharmaceutically acceptable excipient. The pharmaceutical composition is intended to facilitate the administration of the compound or the pharmaceutical combination thereof of the present application to a patient.

As used herein, unless otherwise stated, the terms "contain", "contains", "containing", "include", "includes", "including", "comprise", "comprises", "comprising", or equivalents thereof are open-ended statements and mean that elements, components, and steps that are not specified may be encompassed in addition to those listed.

As used herein, unless otherwise stated, all numbers expressing the amounts of ingredients, measurements, or reaction conditions used herein are to be understood as being modified in all instances by the term "about". The term "about" when connected to a percentage may mean, for example, $\pm 0.1\%$, preferably, $\pm 0.05\%$, and more preferably, $\pm 0.01\%$.

Unless otherwise specified clearly herein, singular terms encompass plural referents, and vice versa. Similarly, unless otherwise specified clearly herein, the word "or" is intended to include "and", and vice versa.

Herein, unless otherwise stated, the term "optional" or "optionally" means that the object or event it modifies is present or absent, or occurs or does not occur, e.g., "a pharmaceutical combination comprises optional anlotinib or a pharmaceutically acceptable salt thereof' means that the pharmaceutical combination may or may not comprise anlotinib or the pharmaceutically acceptable salt thereof.

The "driver-negative non-small cell lung cancer" in the present application refers to a non-small cell lung cancer with no driver mutation. In some embodiments, the driver is EGFR and/or ALK. In some embodiments, the driver-negative non-small cell lung cancer does not comprise EGFR- and/or ALK-mutated non-small cell lung cancer.

The "clinical benefits" in the present application include, but are not limited to: prolonged progression-free survival (PFS), prolonged overall survival (OS), improved objective response rate (ORR), improved disease control rate (DCR), prolonged time to objective response (TTR), and reduced number and/or degree of adverse effects for clinical patients.

All patents, patent applications, and other publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to

these publications herein shall not be construed as an admission that the publications form part of the common knowledge in the art.

## DETAILED DESCRIPTION

For clarity, the present application is further described with the following examples, which are, however, not intended to limit the scope of the present application. All reagents used in the present application are commercially available and can be used without further purification. In the examples, the anti-PD-L1 antibody may be prepared as described in WO2016022630, and after affinity chromatography, an eluate containing the antibody was obtained by conventional antibody purification methods.

**Example 1.** Clinical Trial

Subjects who met the inclusion criteria were randomly assigned to a trial group (squamous cell carcinoma), a trial group (non-squamous cell carcinoma), a control group (squamous cell carcinoma), and a control group (non-squamous cell carcinoma). The sample size ratio of the trial groups and the control groups was 2:1, and the course of treatment was divided into an induction treatment phase (i.e., first treatment phase) and a maintenance treatment phase (i.e., second treatment phase). Every 3 weeks (21 days) was counted as one treatment cycle. The patients first received 4 cycles of induction treatment, and were then subjected to the maintenance treatment phase until loss of clinical benefits, intolerable toxicity, PD upon efficacy assessment, or investigator-assessed ineligibility for further treatment. Efficacy evaluation was performed once every 6 weeks (42 days), and then once every 9 weeks (63 days) after 54 weeks.

1.1 Primary inclusion criteria

1) According to the 8th edition of TNM classification for lung cancer of the International Association for the Study of Lung Cancer and the American Joint Committee on Cancer, patients with histologically or cytologically confirmed locally advanced (stage IIIB/IIIC) or metastatic or recurrent (stage IV) NSCLC that is inoperable and not amenable to curative concurrent radiotherapy and chemotherapy (note: combined lung cancer will be classified by major cell type; the subject is not eligible for enrollment if a small cell component is present).
2) Patients who have not previously received systemic anti-tumor treatment for an advanced or recurrent/metastatic disease. For those who have previously received adjuvant chemotherapy, the time interval between the disease recurrence and the last adjuvant chemotherapy should be at least 6 months; the time interval between the end of previous radiotherapy directed to the chest and the current treatment should be more than 6 months, and the time interval between the palliative radiotherapy directed to parts other than the chest and the current treatment should be more than 7 days.
3) For non-squamous non-small cell lung cancer, patients who have been examined and shown not to have EGFR sensitive mutations or ALK fusions (for squamous non-small cell lung cancer, patients with known EGFR mutations or ALK fusions need to be excluded, and the examination is not mandatory for those with unknown status).
4) Those having at least one measurable lesion according to RECIST 1.1 criteria, which should be clearly defined as progressive if they are located within an area for radiotherapy.
5) Age: 18-75 years; male or female; ECOG score: 0-1 point; expected survival time $\geq$ 3 months.

1.2 Study drug

Anti-PD-L1 antibody hu5G11-hIgG1 injection: 1200 mg of anti-PD-L1 antibody injection (strength: 600 mg/20 mL) was diluted to 250 mL with normal saline, the infusion was performed for 60 $\pm$ 10 min, and the infusion system was rinsed with normal saline immediately after the infusion. The anti-PD-L1 antibody injection was administered once every 3 weeks (21 days) on day 1 (d1) of each cycle (d1/q3w). The longest available time for the injection was no more than 24 months.

Anlotinib hydrochloride capsule (with anlotinib dihydrochloride as an active ingredient): once daily (orally taken before breakfast), 1 capsule (10 mg) each time. The oral administration was performed for 2 consecutive weeks and interrupted for 1 week, that is, 21 days was counted as one treatment cycle, and the drug was administered on days 1-14 of each cycle. In the absence of special circumstances, it is recommended to take the drug at a fixed time every day. If a missed dose occurs during the treatment period and it is confirmed that the time to the next dose is shorter than 12 h, there will be no refills (i.e., 10 mg/qd, d1-14/q3w).

The investigator can adjust the dose of the anlotinib hydrochloride capsule, e.g., to 10 mg or 8 mg, according

to the disease condition, safety, and other aspects.

Tislelizumab injection (trade name: Tevimbra): administered on day 1 of each cycle at a dose of 200 mg, diluted with 9 mg/mL (0.9%) sodium chloride or 50 mg/mL (5%) glucose, and administered by intravenous infusion for more than 30 min once every 3 weeks. The time for the first infusion should be no shorter than 60 min; if it is well tolerated, the time for each subsequent infusion should be no shorter than 30 min. The longest available time for the injection was no more than 24 months.

Paclitaxel injection: administered on day 1 of each cycle at a dose of 175 mg/$m^2$, diluted with normal saline or 5% glucose saline, and administered by intravenous drip for 3 h.

Pemetrexed disodium injection: at a dose of 500 mg/$m^2$, reconstituted and further diluted prior to intravenous infusion with 0.9% sodium chloride injection (without preservatives), and administered on day 1 of each cycle by intravenous drip for more than 10 min.

Carboplatin for injection: at a dose of 5 mg/mL/min AUC, dissolved and diluted with 5% glucose, administered on day 1 of each cycle, and administered by intravenous drip (hydrated as appropriate), with a maximum dose of 800 mg for carboplatin.

The dosage of carboplatin injection needs to be calculated according to the area under the plasma concentration-time curve (AUC) and the endogenous creatinine clearance rate. AUC (mg/mL/min) was taken as 5. Calvert formula for the dose of carboplatin by AUC:

$$\text{Dose of carboplatin (mg)} = \text{set AUC (mg/mL/min)} \times [\text{creatinine clearance rate (mL/min)} + 25]$$

Endogenous creatinine clearance rate was calculated using the Cockcroft formula.

$$\text{Creatinine clearance rate for male (mL/min)} = $$ Fehler!

$$\text{Creatinine clearance rate for female (mL/min)} = $$ Fehler!

Note: serum creatinine concentration in (mg/dL); body weight in Kg.

Recommended calculation formula of the body surface area: adult body surface area ($m^2$) = 0.0061 × height (cm) + 0.0128 × body weight (kg) - 0.1529.

1.3 Administration regimen

The course of treatment is divided into an induction treatment phase (i.e., first treatment phase) and a maintenance treatment phase (i.e., second treatment phase).

The first treatment phase (4 treatment cycles):

Trial group (squamous cell carcinoma):

anti-PD-L1 antibody injection: administered at a dose of 1200 mg on d1 of each cycle by intravenous infusion;
paclitaxel injection: administered on d1 of each cycle by intravenous drip at 175 mg/$m^2$, after an interval of at least 30 min after the administration of the anti-PD-L1 antibody;
carboplatin for injection: administered on d1 of each cycle by intravenous drip at AUC 5 mg/mL/min, immediately after the administration of paclitaxel, with a maximum dose of 800 mg.

Trial group (non-squamous cell carcinoma):

anti-PD-L1 antibody injection: administered at a dose of 1200 mg on d1 of each cycle by intravenous infusion;
pemetrexed disodium injection: administered on d1 of each cycle by intravenous drip at a dose of 500 mg/$m^2$, after an interval of at least 30 min after the administration of the anti-PD-L1 antibody;
carboplatin for injection: administered on d1 of each cycle by intravenous drip at AUC 5 mg/mL/min, after an interval of at least 30 min after the administration of pemetrexed, with a maximum dose of 800 mg.

Control group (squamous cell carcinoma):

tislelizumab injection: administered at a dose of 200 mg on d1 of each cycle by intravenous infusion;

paclitaxel injection: administered on d1 of each cycle by intravenous drip at 175 mg/$m^2$, after an interval of at least 30 min after the administration of tislelizumab;
carboplatin for injection: administered on d1 of each cycle by intravenous drip at AUC 5 mg/mL/min, immediately after the administration of paclitaxel, with a maximum dose of 800 mg.

Control group (non-squamous cell carcinoma):

tislelizumab injection: administered at a dose of 200 mg on d1 of each cycle by intravenous infusion;
pemetrexed disodium injection: administered on d1 of each cycle by intravenous drip at 500 mg/$m^2$, after an interval of at least 30 min after the administration of tislelizumab;
carboplatin for injection: administered on d1 of each cycle by intravenous drip at AUC 5 mg/mL/min, after an interval of at least 30 min after the administration of pemetrexed, with a maximum dose of 800 mg.

Second treatment phase (until loss of clinical benefits, intolerable toxicity, PD upon efficacy assessment, or investigator-assessed ineligibility for further treatment):

Trial group (squamous cell carcinoma):

anti-PD-L1 antibody injection: administered at a dose of 1200 mg on d1 of each cycle by intravenous infusion;
anlotinib hydrochloride capsule: 10 mg each time, once daily, orally taken before breakfast, and administered on d1-d14 of each cycle for 2 weeks and interrupted for 1 week.

Trial group (non-squamous cell carcinoma):

anti-PD-L1 antibody injection: administered at a dose of 1200 mg on d1 of each cycle by intravenous infusion;
pemetrexed disodium injection: administered on d1 of each cycle by intravenous drip at a dose of 500 mg/$m^2$, after an interval of at least 30 min after the administration of the anti-PD-L1 antibody;
anlotinib hydrochloride capsule: 10 mg each time, once daily, orally taken before breakfast, and administered on d1-d14 of each cycle for 2 weeks and interrupted for 1 week.

Control group (squamous cell carcinoma):
tislelizumab injection: administered at a dose of 200 mg on d1 of each cycle by intravenous infusion.
Control group (non-squamous cell carcinoma):

tislelizumab injection: administered at a dose of 200 mg on d1 of each cycle by intravenous infusion;
pemetrexed disodium injection: administered on d1 of each cycle by intravenous drip at 500 mg/$m^2$, after an interval of at least 30 min after the administration of tislelizumab.

1.4 Evaluation criteria

Efficacy evaluation criteria: RECIST 1.1 criteria were used for evaluation, and iRECIST was used for review when subjects first developed progressive disease (PD).
Safety evaluation criteria: NCI-CTC AE 5.0 criteria were used to determine the severity of adverse events.

1.5 Study endpoint

Primary endpoint: progression-free survival (PFS) as evaluated by IRC according to RECIST1.1 criteria.
Secondary endpoints: (1) efficacy endpoints: overall survival (OS), progression-free survival (PFS) as evaluated by investigators according to RECIST1.1 and iRECIST criteria, objective response rate (ORR), disease control rate (DCR), duration of response (DOR), and time to objective response (TTR); (2) safety endpoints: incidence and severity of adverse events (AEs), laboratory test value abnormalities, and severe adverse events (SAEs); (3) quality of life: EORTC quality of life questionnaire (QLQ-C30), lung cancer module (QLQ-LC13), lung cancer symptom scale (LCSS), and EQ-5D assessment PRO; and (4) anti-PD-L1 antibody immunogenicity: incidence of ADA and NAB.
Exploratory endpoints: relationship between PD-L1 expression and efficacy.

1.6 Trial results

Preliminary studies showed that the pharmaceutical combination of the trial group could safely and effectively

treat non-small cell lung cancer. Subjects receiving regimens in the trial group (squamous cell carcinoma) and the trial group (non-squamous cell carcinoma) were all expected to achieve a more significant clinical benefit as compared to those receiving regimens in the control groups. Median PFS and ORR were improved for subjects in both the trial group (squamous cell carcinoma) and the trial group (non-squamous cell carcinoma). By way of example only, the conditions of some of the subjects are shown as follows:

Patient 1, 71 years old, with a history of smoking for more than 30 years, has been suggested to have invasive lung adenocarcinoma through biopsy pathology, has been clinically diagnosed as adenocarcinoma in the right lung middle lobe-hilus, with metastasis to lymph nodes in mediastinal zone 7 and malignant pleural effusion, and has been clinically classified into stage T2bN2M1a (IV). The patient met the inclusion criteria after relevant screening examinations, and then was enrolled into the trial group (non-squamous cell carcinoma). By an imaging examination in the screening period, the target lesion is a mass in the right lung middle lobe-hilus, with a maximum diameter of 54.99 mm. By the date of data statistics, patient 1 had received treatment of more than 12 treatment cycles, with the target lesion of a mass in the right lung lower lobe shrinking to 44 mm, disappearance of enlarged lymph nodes in mediastinal zone 7, disappearance of pleural effusion, and an overall efficacy evaluation of stable disease (SD). The patient had a PFS of more than 9 months. With continued treatment, patient 1 was expected to continue to benefit from this trial protocol.

Patient 2, 66 years old, with a history of smoking for more than 10 years, has been suggested to have invasive lung adenocarcinoma by biopsy pathology, has been clinically diagnosed as lung adenocarcinoma in the basal segment of the left lung lower lobe, with multiple metastases to the left supraclavicular, mediastinal, and peritoneal lymph nodes, and bone metastases to the left ribs, and has been clinically classified into stage T2N3M1a (IV). The patient met the inclusion criteria after relevant screening examinations, and then was enrolled into the trial group (non-squamous cell carcinoma). By an imaging examination in the screening period, the target lesions include a mass in the posterior basal segment of the lung left lower lobe, 4L lymph nodes in the mediastinum, and a left supraclavicular lymph node, with a total diameter of 83.6 mm. After treatment with 2 treatment cycles, the sum of the diameters of the target lesions was reduced to 43.4 mm by about 48%, and the efficacy was evaluated as partial response (PR). By the date of data statistics, patient 2 has received treatment with more than 10 treatment cycles, with the sum of diameters of target lesions reduced to 39 mm, and both mediastinal 4L lymph nodes and left supraclavicular lymph nodes shrinking to less than 10 mm (10 mm being the cut-off for pathological lymph nodes), and with an overall efficacy evaluation of partial response (PR). The patient had a PFS of more than 7 months. With continued treatment, patient 2 was expected to continue to benefit from this trial protocol.

Patient 3, 53 years old, with a history of smoking for 36 years, has been suggested to have invasive lung adenocarcinoma by biopsy pathology, has been clinically diagnosed as adenocarcinoma of the tip segment of the right lung upper lobe and multiple metastases to lymph nodes in the mediastinum and right lung hilum, with obstructive pneumonia, and has been clinically classified into stage T3N3M0 (IIIC). The patient met the inclusion criteria after relevant screening examinations, and then was enrolled into the control group (non-squamous cell carcinoma). By an imaging examination in the screening period, the target lesions include the soft tissue lesion of the right lung upper lobe, lymph nodes in the right lung hilum, and mediastinal lymph nodes, with a total diameter of 116.8 mm. After treatment with 4 treatment cycles, the sum of the diameters of the target lesions was reduced by 50.3%, and the efficacy was evaluated as partial response (PR). However, after treatment with 8 treatment cycles, the sum of the diameters of the target lesions measured was 101 mm, and non-target nodules in the subclavicular region were enlarged, with a comprehensive evaluation of progressive disease (PD). Patient 3 withdrew from the trial with a PFS of 5.8 months.

Those skilled in the art will recognize that the scope of the present application is not limited to the embodiments and examples described above. Instead, various modifications, substitutions, or recombinations can be made without departing from the spirit of the present application, all of which fall within the protection scope of the present application.

## Claims

1. Use of a pharmaceutical combination comprising an anti-PD-L1 antibody and at least one chemotherapeutic drug for preparing a medicament for treating non-small cell lung cancer, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

2. The use according to claim 1, wherein the pharmaceutical combination comprises a first pharmaceutical combination and optionally a second pharmaceutical combination; the first pharmaceutical combination comprises the anti-PD-L1 antibody and at least one chemotherapeutic drug, and the second pharmaceutical combination comprises the anti-PD-L1 antibody, anlotinib or a pharmaceutically acceptable salt thereof, and optionally a chemotherapeutic drug.

3. The use according to claim 2, wherein the first pharmaceutical combination is administered to a patient in need thereof in a first treatment phase, and optionally, the second pharmaceutical combination is administered to the patient in need thereof in a second treatment phase, the second treatment phase being performed sequentially after the first treatment phase;
preferably, the first treatment phase comprises 1 to 10 treatment cycles, preferably 4 to 6 treatment cycles, and most preferably 4 treatment cycles.

4. The use according to claim 3, wherein every 1 week, every 2 weeks, every 3 weeks, or every 4 weeks is counted as one treatment cycle, preferably, every 3 weeks is counted as one treatment cycle.

5. The use according to any one of claims 1-4, wherein the chemotherapeutic drug is selected from the group consisting of one or more of a platinum-based anti-tumor drug, a taxane anti-tumor drug, and an antimetabolite anti-tumor drug; preferably, the chemotherapeutic drug comprises: a platinum-based anti-tumor drug, and at least one selected from the group consisting of a taxane anti-tumor drug and an antimetabolite anti-tumor drug.

6. The use according to claim 5, wherein the platinum-based anti-tumor drug is selected from the group consisting of one or more of cisplatin, carboplatin, nedaplatin, dicycloplatin, picoplatin, oxaliplatin, miriplatin, lobaplatin, triplatin tetranitrate, phenanthriplatin, and satraplatin; the taxane anti-tumor drug is selected from the group consisting of one or more of paclitaxel, paclitaxel liposome, albumin-bound paclitaxel, and docetaxel; the antimetabolite anti-tumor drug is selected from the group consisting of one or more of carmofur, 5-fluorouracil, tegafur, capecitabine, tegafur-gimeracil-oteracil potassium, difuradin, doxifluridine, trifluridine, cytarabine, gemcitabine, azacitidine, ancitabine, mercaptopurine, fludarabine, methotrexate, and pemetrexed.

7. The use according to any one of claims 1-6, wherein the non-small cell lung cancer is non-small cell lung cancer that is inoperable, and/or not amenable to curative concurrent radiotherapy and chemotherapy, and/or advanced, and/or recurrent, and/or metastatic;

   the patient with the non-small cell lung cancer has not previously received systemic anti-tumor treatment, or the patient has previously received treatment with a chemotherapy regimen, or the patient has previously received neoadjuvant and/or adjuvant chemotherapy;
   the non-small cell lung cancer is driver-negative non-small cell lung cancer;
   the non-small cell lung cancer is EGFR and/or ALK wild-type non-small cell lung cancer; or
   the non-small cell lung cancer is squamous non-small cell lung cancer, non-squamous non-small cell lung cancer, or lung adenocarcinoma.

8. The use according to any one of claims 2-6, wherein the chemotherapeutic drug in the first pharmaceutical combination is one or more of carboplatin, pemetrexed, and paclitaxel; preferably, the chemotherapeutic drug in the first pharmaceutical combination is carboplatin and at least one selected from the group consisting of pemetrexed and paclitaxel.

9. The use according to claim 8, wherein the anti-PD-L1 antibody, carboplatin, and paclitaxel comprised in the first pharmaceutical combination are administered to a patient in need thereof at the following doses, respectively:

   the anti-PD-L1 antibody being administered at a daily dose of 600-2400 mg, paclitaxel being administered at a daily dose of 100-200 mg/m$^2$, and carboplatin being administered at a dose of AUC 1-10 mg/mL/min with a daily of no more than 800 mg; or
   the anti-PD-L1 antibody, carboplatin, and pemetrexed comprised in the first pharmaceutical combination are administered to the patient in need thereof at the following doses, respectively: the anti-PD-L1 antibody being administered at a daily dose of 600-2400 mg, pemetrexed being administered at a daily dose of 200-800 mg/m$^2$, and carboplatin being administered at a dose of AUC 1-10 mg/mL/min with a daily of no more than 800 mg.

10. The use according to any one of claims 2-9, wherein the anti-PD-L1 antibody and anlotinib or the pharmaceutically acceptable salt thereof comprised in the second pharmaceutical combination are administered to the patient in need

thereof at the following doses, respectively: the anti-PD-L1 antibody being administered at a daily dose of 600-2400 mg, and anlotinib or the pharmaceutically acceptable salt thereof being administered at a daily dose of 6-12 mg; or the anti-PD-L1 antibody, anlotinib or the pharmaceutically acceptable salt thereof, and pemetrexed comprised in the second pharmaceutical combination are administered to the patient in need thereof at the following doses, respectively: the anti-PD-L1 antibody being administered at a daily dose of 600-2400 mg, anlotinib or the pharmaceutically acceptable salt thereof being administered at a daily dose of 6-12 mg, and pemetrexed being administered at a daily dose of 200-800 $mg/m^2$.

**11.** The use according to any one of claims 2-10, wherein the anti-PD-L1 antibody and at least one chemotherapeutic drug, and optionally anlotinib or the pharmaceutically acceptable salt thereof are each present in the form of a pharmaceutical composition and can be administered simultaneously, successively, or sequentially.

**12.** The use according to any one of claims 2-11, wherein every 3 weeks is counted as one treatment cycle, the anti-PD-L1 antibody is administered on day 1 of each cycle, and optionally, anlotinib or the pharmaceutically acceptable salt thereof is administered on days 1-14 of each cycle.

**13.** A method for treating non-small cell lung cancer, comprising: administering to a patient in need thereof a therapeutically effective amount of an anti-PD-L1 antibody and at least one chemotherapeutic drug, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

**14.** The method according to claim 13, comprising: administering a first pharmaceutical combination to the patient in need thereof in a first treatment phase; and optionally administering a second pharmaceutical combination to the patient in need thereof in a second treatment phase, the second treatment phase being performed sequentially after the first treatment phase, wherein the first pharmaceutical combination comprises the anti-PD-L1 antibody and at least one chemotherapeutic drug, and the second pharmaceutical combination comprises the anti-PD-L1 antibody, anlotinib or a pharmaceutically acceptable salt thereof, and optionally a chemotherapeutic drug.

**15.** A pharmaceutical combination for use in treating non-small cell lung cancer, comprising an anti-PD-L1 antibody and at least one chemotherapeutic drug, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

**16.** The pharmaceutical combination for use in treating non-small cell lung cancer according to claim 15, wherein the pharmaceutical combination comprises a first pharmaceutical combination and optionally a second pharmaceutical combination, wherein the first pharmaceutical combination comprises the anti-PD-L1 antibody and at least one chemotherapeutic drug, and the second pharmaceutical combination comprises the anti-PD-L1 antibody, anlotinib or a pharmaceutically acceptable salt thereof, and optionally a chemotherapeutic drug.

**17.** The pharmaceutical combination for use in treating non-small cell lung cancer according to claim 15, wherein the pharmaceutical combination comprises: (1) the anti-PD-L1 antibody and a chemotherapeutic drug comprising a platinum-based anti-tumor drug administered to a patient in need thereof, and sequentially administered (2) the anti-PD-L1 antibody, anlotinib or a pharmaceutically acceptable salt thereof, and optionally a chemotherapeutic drug.

**18.** The use according to any one of claims 1-12, the method according to claim 13 or 14, or the pharmaceutical combination according to any one of claims 15-17, wherein the anti-PD-L1 antibody comprises the following amino acid sequences: a heavy chain CDR1 region selected from the group consisting of SEQ ID NO: 1 and SEQ ID NO:

4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12;
preferably, the anti-PD-L1 antibody comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

**19.** The use according to any one of claims 1-12, or the method according to claim 13 or 14, or the pharmaceutical combination for use in treating non-small cell lung cancer according to any one of claims 15-17, wherein the anti-PD-L1 antibody comprises the following amino acid sequences: a heavy chain variable region having at least 80% homology to an amino acid sequence set forth in SEQ ID NO: 13 or SEQ ID NO: 14; and a light chain variable region having at least 80% homology to an amino acid sequence set forth in SEQ ID NO: 15 or SEQ ID NO: 16.

**20.** The use according to any one of claims 1-12, the method according to claim 13 or 14, or the pharmaceutical combination for use in treating non-small cell lung cancer according to any one of claims 15-17, wherein the anti-PD-L1 antibody comprises: a heavy chain variable region selected from the group consisting of heavy chain variable regions of humanized antibodies hu13C5-hIgG1, hu13C5-hIgG4, hu5G11-hIgG1, and hu5G11-hIgG4; and a light chain variable region selected from the group consisting of light chain variable regions of humanized antibodies hu13C5-hIgG1, hu13C5-hIgG4, hu5G11-hIgG1, and hu5G11-hIgG4.

**INTERNATIONAL SEARCH REPORT**

International application No. **PCT/CN2022/135865**

**A. CLASSIFICATION OF SUBJECT MATTER**

C07K16/28(2006.01)i;A61P35/00(2006.01)i;A61K39/395(2006.01)i

According to International Patent Classification (IPC) or to both national classification and IPC

**B. FIELDS SEARCHED**

Minimum documentation searched (classification system followed by classification symbols)

C07K, A61P, A61K

Documentation searched other than minimum documentation to the extent that such documents are included in the fields searched

Electronic data base consulted during the international search (name of data base and, where practicable, search terms used)

VEN, CNABS, PubMed, ISI Web of Science, VCN, CNKI, 万方, 百度学术, 读秀, STN, GenBank, 中国专利生物序列检索系统: PD-L1, PDL1, CD274, 单克隆抗体, 单抗, monoclonal antibody, 化疗药物, chemotherayp drug, 安罗替尼, anlotinib, 非小细胞肺癌, NSCLC, SEQ ID NOs: 1-17.

**C. DOCUMENTS CONSIDERED TO BE RELEVANT**

| Category* | Citation of document, with indication, where appropriate, of the relevant passages | Relevant to claim No. |
|---|---|---|
| X | WO 2020249018 A1 (CHIA TAI TIANQING PHARMACEUTICAL GROUP NANJING SHUNXIN PHARMACEUTICAL CO., LTD. ET AL.) 17 December 2020 (2020-12-17) claims 1-21, description， page 1 paragraph 8 - page 10 last paragraph | 1-20 |
| Y | CN 107001463 A (CROWN BIOSCIENCE, INC. (TAICANG) ET AL.) 01 August 2017 (2017-08-01) claims 1-46, description sequence listings 88-140 | 1-20 |
| Y | WO 2020187152 A1 (CHIA TAI TIANQING PHARMACEUTICAL GROUP CO., LTD. ET AL.) 24 September 2020 (2020-09-24) claims 1-19, and description, page 1, paragraph 7-page 9, paragraph 7 | 1-20 |
| Y | HAN, Baohui et al. "Anlotinib as a third-line therapy in patients with refractory advanced non-small-cell lung cancer: a multicentre, randomised phase II trial (ALTER0302)" *British Journal of Cancer,* Vol. 118, 13 February 2018 (2018-02-13), pp. 654-661 | 1-20 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 July 2023** | **21 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/135865**

**C. DOCUMENTS CONSIDERED TO BE RELEVANT**

| Category* | Citation of document, with indication, where appropriate, of the relevant passages | Relevant to claim No. |
|---|---|---|
| A | WO 2020156500 A1 (CHIA TAI TIANQING PHARMACEUTICAL GROUP CO., LTD. ET AL.) 06 August 2020 (2020-08-06)<br>entire document | 1-20 |
| A | CN 113018429 A (CHIA TAI TIANQING PHARMACEUTICAL GROUP NANJING SHUNXIN PHARMACEUTICAL CO., LTD. ET AL.) 25 June 2021 (2021-06-25)<br>entire document | 1-20 |
| A | CN 112168961 A (CHIA TAI TIANQING PHARMACEUTICAL GROUP NANJING SHUNXIN PHARMACEUTICAL CO., LTD. ET AL.) 05 January 2021 (2021-01-05)<br>entire document | 1-20 |

Form PCT/ISA/210 (second sheet) (July 2022)

**INTERNATIONAL SEARCH REPORT**

International application No.
**PCT/CN2022/135865**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2022/135865**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13-14, 18-20 (in part)**
because they relate to subject matter not required to be searched by this Authority, namely:

Claims 13-14 and 18-20 (in part) relate to a method for treating a tumor and/or cancer, belonging to a method for treating a living human or animal body, and belonging to the subject matter as defined in PCT Rule 39.1(iv) that does not warrant a search conducted by the international searching authority. The international search was conducted on the basis of a use of a PD-L1 monoclonal antibody and a chemotherapeutic drug in the preparation of a drug for treating non-small cell lung cancer.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/135865**

| Patent document cited in search report | Publication date (day/month/year) | Patent family member(s) | Publication date (day/month/year) |
|---|---|---|---|
| WO 2020249018 A1 | 17 December 2020 | None | |
| CN 107001463 A | 01 August 2017 | US 2017204184 A1 | 20 July 2017 |
| | | US 10435470 B2 | 08 October 2019 |
| | | MX 2017001597 A | 17 November 2017 |
| | | BR 112017002234 A2 | 24 July 2018 |
| | | AU 2015301126 A1 | 23 February 2017 |
| | | AU 2015301126 B2 | 11 March 2021 |
| | | KR 20170039706 A | 11 April 2017 |
| | | KR 102476226 B1 | 12 December 2022 |
| | | CA 2956399 A1 | 11 February 2016 |
| | | SG 11201700687 TA | 27 February 2017 |
| | | RU 2017106804 A | 06 September 2018 |
| | | RU 2017106804 A3 | 27 August 2019 |
| | | RU 2722212 C2 | 28 May 2020 |
| | | RU 2722212 C9 | 23 July 2020 |
| | | WO 2016022630 A1 | 11 February 2016 |
| | | AU 2021203593 A1 | 01 July 2021 |
| | | US 2020031935 A1 | 30 January 2020 |
| | | US 11111300 B2 | 07 September 2021 |
| | | JP 2017523786 A | 24 August 2017 |
| | | JP 6909153 B2 | 28 July 2021 |
| | | IL 250415 A0 | 30 March 2017 |
| | | IL 250415 B | 30 September 2020 |
| | | US 2021371531 A1 | 02 December 2021 |
| | | SG 10201901057 UA | 28 March 2019 |
| | | JP 2020141672 A | 10 September 2020 |
| | | JP 7144477 B2 | 29 September 2022 |
| | | ZA 201700785 B | 28 July 2021 |
| | | EP 3177649 A1 | 14 June 2017 |
| | | EP 3177649 A4 | 25 April 2018 |
| WO 2020187152 A1 | 24 September 2020 | EP 3939610 A1 | 19 January 2022 |
| | | CA 3133141 A1 | 24 September 2020 |
| | | US 2022175759 A1 | 09 June 2022 |
| | | AU 2020242144 A1 | 04 November 2021 |
| WO 2020156500 A1 | 06 August 2020 | None | |
| CN 113018429 A | 25 June 2021 | None | |
| CN 112168961 A | 05 January 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107001463 A
- WO 2016022630 A